# EUROPEAN PATENT SPECIFICATION

(11) **EP 3 747 490 B1**
(45) Date of publication and mention of the grant of the patent: **21.02.2024**
(21) Application number: 20171749.3
(22) Date of filing: 23.03.2015
(51) Int. Cl.: A61M 16/00, A61M 16/04

(54) **APPARATUS FOR IMPROVED ASSISTED VENTILATION**
VORRICHTUNG FÜR VERBESSERTE UNTERSTÜTZTE BEATMUNG
APPAREIL DE VENTILATION ASSISTÉE AMÉLIORÉE

(30) Priority: 21.03.2014 US 201461969043 P
(43) Date of publication of application: 09.12.2020
(62) Divisional of application: 15765519.2
(73) Proprietor: CoLabs Medical, Inc., Morgan Hill, CA 95038 (US)
(72) Inventor: NOLAN, Clay, Carmel, CA 93921 (US)
(74) Representative: J A Kemp LLP

(56) References cited:
- WO-A1-2009/099380
- WO-A1-2011/154499
- WO-A1-2013/086134

## Description

### CROSS-REFERENCE TO RELATED APPLICATIONS

The present is a non-provisional application of US Provisional Application 61/969,043 filed March 21, 2014 and is a continuation-in-part of U.S. Patent Application 14/296,298 filed June 4,2014 which is a continuation of U.S. Patent Application 13/659,699 filed October 24, 2012 which claims benefit to U.S. Provisional Application No. 61/569,169 filed December 9, 2011.

### BACKGROUND OF THE INVENTION

Intubation is the placement of a tube of an intubation device into an airway lumen of the body of a patient to provide assisted ventilation of the lungs to maintain a supply of oxygen to the blood in those cases where the patient is unable to breathe on his or her own. Intubation in cases of respiratory distress involves the placement of a tube into the trachea of the patient. Tracheal intubation also involves the positioning of an endotracheal tube into a patient's trachea through the vocal cords, so the caregiver must also be careful to avoid injuring the vocal cords. In many cases, care must be taken when intubating a patient since improper placement of the tube can result in additional harm to the patient. For example, many conventional intubation devices rely on an inflatable cuff that forms a seal against the lumen wall to maintain a position of the tube within the lumen. Over-inflation of the cuff, can cause internal bleeding in the patient Another significant problem is that extreme care must be taken to avoid positioning the intubation tube within the esophagus rather than the trachea. In such cases, with conventional devices, the first responder or medical practitioner cannot properly ventilate the patient and the patient can suffer further injury.

Even properly trained medical caregivers and first responders must proceed with caution during intubation to avoid misplacement of the intubation device or to avoid unwanted insertion errors and risk of injury. Delay and/or misplacement of the endotracheal tube, such as misplacement of the endotracheal tube into the esophagus, can potentially result in neurological damage or death. Improper positioning of the endotracheal tube also can compromise airway protection or result in inadequate ventilation. It is therefore imperative to intubate a patient quickly and position the endotracheal tube correctly when a medical condition arises.

To reduce the risk of complications during intubation, the caregiver, whether a first responder, such as an emergency medical technician, paramedic, or a nurse or physician must proceed as quickly as possible yet with caution to avoid the potential complications. In addition, a first responder must often attempt to intubate the patient in a less than desirable location such as a bathroom, restaurant, or other area not conducive to providing proper medical treatment and care.

Assisted ventilation in cases of cardiac arrest also requires prompt and accurate placement of an intubation device within the trachea so that chest compressions can occur. In such cases, intubation allows for ventilation of the lungs and a supply of oxygen to the blood while chest compressions provide circulation of the blood.

The American Heart Association's protocols for cardio pulmonary resuscitation (CPR) previously required pausing after every fifteen chest compressions to allow for two ventilations. The American Heart Association's 2010 protocols decreased the frequency of ventilations such that chest compressions are to be paused after every thirty compressions to allow for two ventilations. It is believed that the main reasons supporting the change in protocol are: 1) reduce the amount of intra-thoracic pressure associated with positive pressure ventilations since positive pressure ventilations decrease the efficiency of the heart; and 2) to minimize the interruptions of chest compressions to maintain constant arterial pressure. Accordingly, now most caregivers only simultaneously ventilate the patient and provide compressions if the patient is properly intubated.

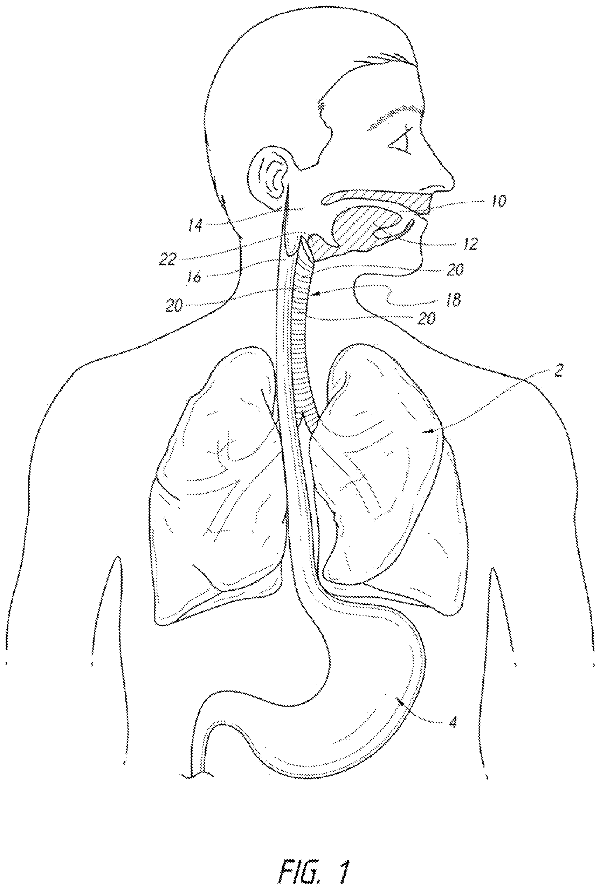

FIG. 1 provides a partial view of a patient's oral cavity **10,** tongue **12** and pharynx **14** where the pharynx **14** is the membrane-lined cavity at the rear of the oral cavity **10.** The pharynx **14** includes openings of the esophagus **16** and trachea **18.** As shown, the openings to the esophagus **16** and trachea **18** are adjacent to one another. When a medical caregiver attempts to intubate a patient, the caregiver shall attempt to position the intubation device within the trachea **18** to provide oxygen to the lungs **2.** As noted above, the caregiver shall attempt to avoid positioning the intubation device within the esophagus **16** and in doing so often must proceed slowly and with caution to avoid causing undesired trauma to vocal cords or other structures within the body.

The wall of the esophagus **16** is composed of striated and smooth muscle. Since the esophagus **16** relies on peristalsis to move food downward towards the stomach, the walls of the esophagus **16** are naturally compliant and do not have any structural reinforcement. The trachea **18,** on the other hand, is relatively stronger and is naturally designed not to collapse given its function of transporting air to the bronchi and lungs **2.** The wall of the trachea **18** includes a number of cartilaginous semicircular rings **20** that prevent the trachea **18** from collapsing. The trachea **20** lies anteriorly to the esophagus **16** where the openings of the esophagus **16** and trachea are separated by a tiny flap, the epiglottis **22.** The epiglottis **22** protects the trachea when the individual swallows food or other substances.

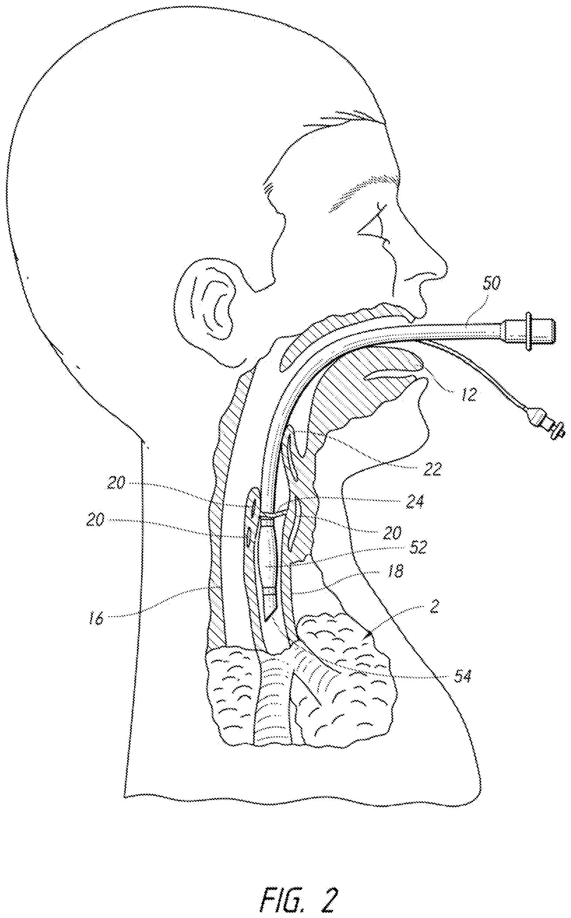

FIG. 2 illustrates a conventional device **50** used for intubating a patient. As shown, the device **50** is inserted through the mouth and oral cavity into the trachea **18.** The caregiver must navigate the device **50** into the trachea **18** rather than the esophagus while traversing the epiglottis **22** and vocal cords **24.** The caregiver must take particular care to avoid causing damage to the vocal cords **24.** Once properly positioned, the caregiver can optionally inflate **52** a balloon on the device **50** to anchor the device within the trachea **18.** After the caregiver confirms placement of the device **50,** ventilation of the patient can take place.

Presently, the Combitube, supplied by Nellcor, is commonly used for airway management. The Combitube, also known as a double-lumen airway, is a blind insertion airway device (BIAD) used by first responders as well as in an emergency room setting. The Combitube is intended to allow for tracheal intubation of a patient in respiratory distress by use of a cuffed, double-lumen tube. The double lumen tube is inserted into the patient's airway to allow for ventilation of the patient's lungs. Inflation of the cuff allows the device to function similarly to an endotracheal tube and usually closes off the esophagus, allowing ventilation and preventing pulmonary aspiration of gastric contents.

However, placement of traditional intubation devices is very difficult due to the risk of improperly positioning the device. The risk of a device being improperly positioned can be fatal if not recognized. The conventional devices described above require positioning by an individual that is well trained in positioning such devices. Furthermore, even well trained individuals must proceed with caution when placing conventional devices.

In addition, there remains a need to improve timing of air delivery during artificial ventilations of a patient. This need especially remains where the patient is experiencing distress and requires both ventilation for oxygen and chest compression to reestablish blood circulation. Presently, if the act of artificially ventilating the individual (e.g., through assisted ventilation or mouth-to-mouth) and providing chest compressions is not timed, such as during normal CPR, normal artificial ventilation of the individual can work against the effectiveness of the compression. For instance, assisted ventilation by repeatedly delivering a large bolus of air can raise the pressure within the thoracic cavity and increase resistance by raising pressure on the heart. This back pressure can prevent the heart and lungs from filling with blood. As a result, impeding the ability of the heart and lungs to fill with blood, makes the chest compression less effective as a lower volume of blood is circulated after the compression.

There remains a need for a ventilation device and/or system that can effectively ventilate individuals and can be effectively positioned with minimal training required by the caregiver. In addition, there remains a need for such ventilation devices and methods to optimize the effect of providing assisted ventilation with chest compressions to circulate oxygenated blood within an individual.

WO 2013/086134A discloses devices and methods for allowing for improved assisted ventilation of a patient. The methods and devices provide a number of benefits over conventional approaches for assisted ventilation. For example, the methods and devices permit blind insertion of a device that can allow ventilation regardless of whether the device is positioned within a trachea or an esophagus.

### SUMMARY OF THE INVENTION

The invention is defined in claim 1. Further aspects and preferred embodiments are defined in the dependent claims. Any aspects, embodiments and examples of the present disclosure which do not fall under the scope of the appended claims do not form part of the invention and are merely provided for illustrative purposes.

The present disclosure includes devices and methods allowing for improved assisted ventilation of a patient. The methods and devices provide a number of benefits over conventional approaches for assisted ventilation. For example, the methods and devices described herein permit blind insertion of a device that can allow ventilation regardless of whether the device is positioned within a trachea or an esophagus. Some variations of the devices and methods allow minimally trained bystanders and laypersons to place an advanced airway for assisted ventilation. The devices described herein can be designed such that a single size can accommodate a variety of patient sizes thereby reducing the number of devices of varying sizes that must be kept in inventory. Additionally, having devices that can accommodate a wide range of individuals reduces the need of a first responder to assess the anatomic features of a patient prior to acting on the patient. In patients undergoing cardiac distress, delivery of large boluses of air during CPR can result in hyperventilating the patient, which can decrease the effectiveness of CPR. Elevated intrathoracic pressure can ultimately reduce the effectiveness of chest compressions. Variations of the current device and methods allow for controlled ventilation, which avoids hyperventilation.

In another variation, the devices described in the present disclosure allow for improved assisted ventilating an individual. For example, a variation of the method includes inserting a ventilation device into the individual by advancing a working end of the ventilation device within a body passage of the individual where the working end includes a far opening fluidly coupled to a first lumen and a medial opening fluidly coupled to a second lumen; drawing suction through the opening and attempting to hold a vacuum through the first lumen and the far opening for a pre-determined period of time; automatically ventilating the individual through the second lumen upon detecting the vacuum during the pre-determined period of time and maintaining suction to maintain the vacuum; and automatically ventilating the individual through the first lumen upon failure to detect the vacuum during the pre-determined period of time; where automatically ventilating the individual occurs at a pre-determined timing; measuring a condition of a thoracic cavity to determine a change in the thoracic cavity; and altering a timing of automatically ventilating of the individual upon detecting the change in the condition of the thoracic cavity.

The present disclosure also includes a system artificially ventilating an individual, the system comprising: a ventilation device configured for insertion within a respiratory opening of the individual and having a working end for positioning within a body passageway of the individual, the ventilation device having a pressure sensor configured to detect pressure changes within the body passage; and the ventilation device having a control system configured to deliver a bolus of air into an airway the individual at a pre-determined rate until detection of the pressure change within the body passageway, whereafter the control system is configured to alter the pre-determined rate by delaying delivery of the bolus of air until the sensor detects the pressure change in the air within the body passage, where the pressure change within the body passage results from a chest compression.

Measuring the condition of the thoracic cavity can include measuring the condition of the thoracic cavity using the ventilation device or measuring a change in a compression of a chest of the patient. For example, measuring the change of the compression of the chest of the patient comprises observing a force applied to the ventilation device by the body passageway. Alternatively, measuring the change of the compression of the chest of the patient comprises observing a deflection of the chest of the patient using one or more sensors on the chest.

In an additional variation, measuring the condition of the thoracic cavity comprises measuring a state of air flow within the thoracic cavity. Such measuring can be performed using a sensor on the ventilation device and where measuring the state of air flow within the thoracic cavity comprises detecting airflow, pressure, and/or volume using the sensor. Moreover, the sensor can monitor a direction of air flow.

The timing of the artificial ventilation can be altered by using the sensor to determine when a pressure in the body passageway increases and delivering air for automatically ventilating the individual when the pressure in the body passageway increases, or at least before the decrease, the pressure of the ventilations acts like and internal chest compression, then the recoil of the chest draws the air into the lungs.

The methods can further include providing a feedback based on measuring the condition of the thoracic cavity. Such feedback can include information regarding the compression, where the information is selected from a phase, rate, efficiency, depth, and timing. The feedback can also be based on measuring the condition of the thoracic cavity comprises measuring a quality of the chest compression by determining a change in a volume of air in the thoracic cavity. In some variation, the feedback comprises information to increase or decrease a compression applied to a chest of the patient.

Variations of the method include altering the timing of the ventilation to initiate automatic ventilating of the individual when a pressure increases, decreases, or reaches a maximum pressure in the body passageway. The method can further comprise continuing measuring the condition of the thoracic cavity to determine the change in the thoracic cavity after altering the timing of automatically ventilating of the individual and reverting to automatically ventilating the individual at the predetermined timing upon failure to detect the change in the thoracic cavity.

In an additional variation, the method can further include adjusting the ventilating device to suspend automatically ventilating the individual and manually ventilating the individual while maintaining suction to maintain the vacuum if the vacuum is detected.

The methods described herein can include a mask that is slidably positioned along the ventilation device and where the mask can be pressed against the individual with a manual actuator or trigger to isolate the respiratory opening of the individual from an external atmosphere. Typically, the mask will not be sealed against the patient during the automatic assisted ventilation. Therefore, during CPR the system is open. Sealing the mask against the patient and initiating a manual trigger can close the system and administering a bolus of air to manually ventilate the patient.

The methods described herein can further include electrically stimulating a heart of the individual using the ventilation device.

In another variation, the method of artificially ventilating an individual can comprise inserting a ventilation device within a respiratory opening of the individual and positioning a working end of the ventilation device within a body passageway of the individual, the ventilation device having a pressure sensor configured to detect pressure changes within the body passage; delivering a bolus of air into an airway the individual at a pre-determined rate; altering the pre-determined rate by delaying delivery of the bolus of air when the sensor detects a pressure change in the air within the body passage, where the pressure change within the body passage results from a chest compression.

The method can further comprise delivering the bolus of air into the airway of the individual occurs after a pre-determined delay. In some variations, the sensor is configured to intermittently detect pressure changes within the body passageway.

In another variation, the method further comprises, after altering the pre-determined rate, the ventilation device resumes delivering the bolus of air at the pre-determined rate if the pressure sensor fails to detect a pressure range within a first period of time.

In another variation, the method for ventilating an individual includes inserting a ventilation device within a respiratory opening of the individual and advancing a working end of the ventilation device within a body passageway of the individual, where the working end includes a first opening fluidly coupled to a first lumen and a second opening fluidly coupled to a second lumen, where the second opening is located along the ventilation device proximal to the first opening; drawing suction through the first opening to induce collapse of the body passageway and maintaining the suction for a period of time; monitoring a fluid parameter to determine whether the body passageway collapses; automatically ventilating the individual through the second lumen upon detecting collapse of the body passageway and maintaining suction to maintain the collapse of the body passageway; and automatically ventilating the individual through the first lumen upon failure to detect collapse of the body passageway; wherein delivery of a bolus of air during automatically ventilating the individual occurs at a first timing; measuring a condition of a thoracic cavity to determine a change in the thoracic cavity; and altering the timing of the delivery of the bolus of air during automatically ventilating the individual upon detecting the change in the condition thoracic cavity.

In another variation, the present disclosure includes a method for artificially ventilating an individual by coupling a ventilation device to a respiratory opening of a respiratory passage of the individual, positioning a pressure sensor in fluid communication with the respiratory passage, the pressure sensor configured to detect pressure changes within the respiratory passage; delivering a bolus of air into the respiratory passage of the individual at a pre-determined rate; and altering the pre-determined rate by delaying delivery of the bolus of air until the sensor detects a pressure change in the air within the respiratory passage, where the pressure change within the respiratory passage results from a chest compression. Such a method can include any device, including conventional ventilation devices.

The present disclosure also includes a system for artificially ventilating an individual using a source of oxygen, the system comprising: a ventilation device having a pressure sensor configured to detect pressure changes within the body passage, the pressure sensor being positioned on a portion of the device configured to be inserted into a body passageway of the individual; a controller configured to deliver a bolus of air into an airway the individual at a pre-determined rate, where the controller is configured to monitor the pressure sensor and upon detecting a pressure change, the controller alters the pre-determined rate by delaying delivery of the bolus of air.

The present disclosure also includes devices for ventilating an individual. In one example such a device comprises a tubular member having at least a first and second lumen, where the first lumen is fluidly coupled to a first opening located distally relative to a medial opening, where the medial opening is fluidly coupled to the second lumen, where the first opening and medial opening are each fluidly isolated within the tubular member; the tubular member being configured to measure a condition of a body lumen to determine a change in a thoracic cavity of the individual; a control system having a suction source and a gas supply lumen, the control system having a valve configured to fluidly couple the gas supply lumen to either the first lumen or to the second lumen; the control system also capable of drawing suction from the suction source through the first opening and first lumen, where the control system is configured to monitor the first lumen for a vacuum to indicate collapse of the body passageway and formation of a seal at the first opening; where the control system is further configured to selectively form a ventilation path from the supply lumen to the first lumen or second lumen by selecting the first lumen as the ventilation path if collapse of the body passageway is not detected; and selecting the second lumen as the ventilation path if collapse of the body passageway is detected; where the control system is configured to automatically ventilate the individual through the ventilation path at a first rate; and where the control system is further configured to alter the first rate upon detecting the condition of the body lumen.

The system and methods described herein can be compatible with devices that monitor the concentration or partial pressure of carbon dioxide (CO2) in the respiratory gases (capnography). Primarily such devices are monitoring tool for use during anesthesia and intensive care that monitor expiratory CO2 are of interest when rebreathing systems are being used. The ability to integrate the ventilation systems described herein with such capnography systems allows for improved patient care. Furthermore, the systems and methods described herein can be compatible with equipment found in emergency vehicles such as oxygen supplies and/or power supplies. In some variations, the system of the present disclosure can also provide audio or even video (through use of a display screen) instructions to ensure proper operation in those situations where the system may be used by first responders that are not trained emergency personnel.

### BRIEF DESCRIPTION OF THE DRAWINGS

The invention is best understood from the following detailed description when read in conjunction with the accompanying drawings. It is emphasized that, according to common practice, the various features of the drawings are not to-scale. On the contrary, the dimensions of the various features are arbitrarily expanded or reduced for clarity. Also for purposes of clarity, certain features of the invention may not be depicted in some of the drawings. Included in the drawings are the following figures:
FIG. 1 provides a partial view of a patient's oral cavity, tongue, pharynx as well as esophagus and trachea.
FIG. 2 illustrates one example of a conventional device as used to intubate a patient.
FIG. 3 illustrates various components of an example of an improved ventilation system.
FIGS. 4A to 4C illustrate a partial sectional view of a working end of an improved ventilation device.
FIGS. 5A to 5E show a representation of the process of ventilating a patient using an improved ventilation device.
FIGS. 6A to 6C show additional variations of a working end of a ventilation device.
FIG. 7 illustrates a schematic of an electrically powered system.
FIG. 8A shows an example of a component schematic for a pneumatically driven system as described herein.
FIG. 8B provides a component listing for the schematic of FIG. 8A.
FIG. 8C shows a listing of various modes for the system.
FIGS. 8D to 8M illustrates various flow paths for the various modes of operation.
FIG. 9A illustrates another variation of a device useful for providing assisted ventilation with improved outcomes by monitoring a condition of the thoracic cavity.
FIGS. 9B to 9C show a partial isometric view and partial cross sectional views of the mask and trigger used to close the system and initiate manual ventilation.
FIGS. 10A and 10B illustrate examples of the working end of the device when inserted into a body passageway of an individual and monitoring a condition of the thoracic cavity.
FIGS. 11A and 11B illustrate a variation of a system for artificially ventilating an individual using a source of oxygen, such as those described herein.
FIG. 11C shows an external device being used to control the ventilator described herein.
FIG. 12 provides a schematic of a control system relying on the gas supply to provide a source for both ventilation and suction.

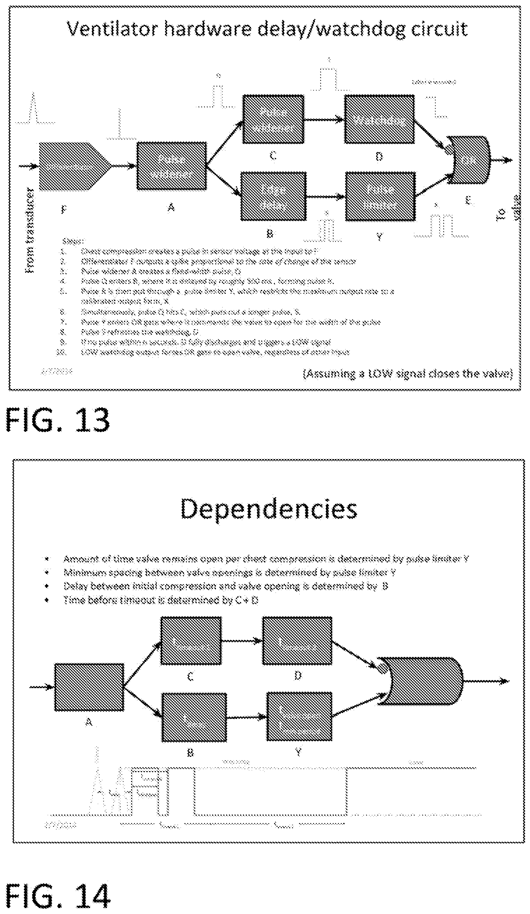

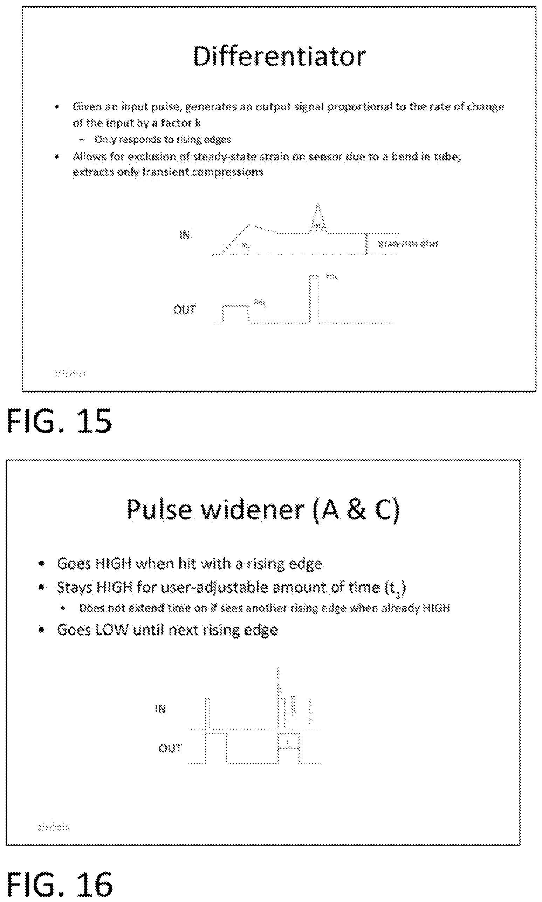

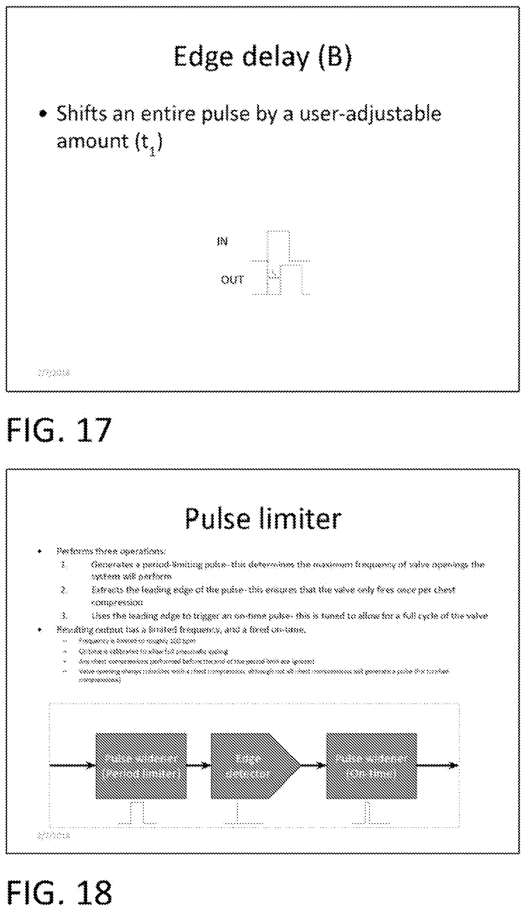

FIGS. 13-22 illustrate an example of the circuitry for sensing the phase, rate, depth and effectiveness of a chest compression with a resistor placed on the tube of an airway placed in the patient's mouth, trachea or esophagus

### DETAILED DESCRIPTION OF THE INVENTION

Before the devices, systems and methods of the present invention are described, it is to be understood that this invention is not limited to particular therapeutic applications and implant sites described, as such may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. The terms "proximal", "distal", "near" and "far" when used indicate positions or locations relative to the user where proximal refers to a position or location closer to the user and distal refers to a position or location farther away from the user.

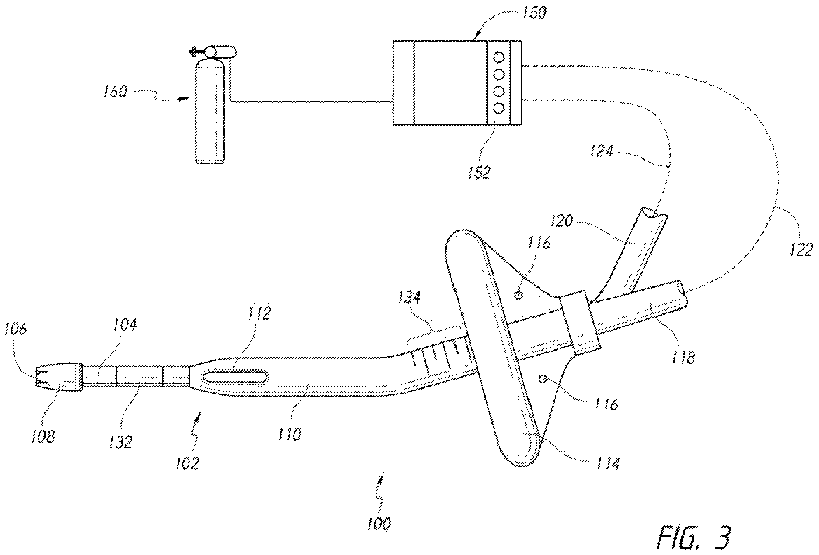

FIG. 3 illustrates various components of an example of an improved system according to the present disclosure. As shown, the ventilation device **100** includes a working end **102** that is inserted into a patient. The working end can include a distal tubing **104** that contains a first lumen (not shown), which extends through a distal opening **106** of the ventilation device **100** and is in fluid communication with a control unit (also called a ventilator) **150** and/or supply source **160** via one or more proximal tubes **118.** The control unit **150** can also include an apparatus designed to provide suction as well as a collection canister. In operation, the control unit **150** directs suction or applies a vacuum through a first fluid path **122,** which in turn causes a suction or negative pressure at the distal opening **106.** The source **160** can comprise oxygen, air, or any other gas that is desired for ventilation of delivery into the lungs. The source **160** can be nested within physical construct of the controller **150.** However, the source **160** can be optional so that the controller ventilates the patient only using ambient air.

The control unit **150** maintains the device **100** in this state for a set period of time and monitors the parameters of the pressure or flow parameters within the first lumen to determine whether to ventilate through the first or second. The example illustrated in FIG. 3 also includes a hub **108** with one or more features that aid in proper functioning of the device. Such features are described in detail below. Furthermore, the distal opening **106** can include any number of ports at the distal end of the device so long as the ports are in a fluid path with the first lumen. Likewise, the medial opening **112** can comprise any number of openings as long as those openings are in fluid communication with the second lumen. In addition, variations of the device can also be inserted through a nasal opening rather than a mouth.

The ventilation device **100** further includes a proximal tubing **110** that houses a second lumen (not shown) that exits the device **100** at a medial opening **112.** As discussed below, distal opening and first lumen are fluidly isolated from the medial opening and second lumen through the working end of the device **102** to the control unit **150.** This fluid isolation allows the control unit **150** to determine which lumen to use to ventilate the patient. The control unit directs flow through a second fluid path **124** that is fluidly coupled to the second lumen and medial opening **112** when the device is positioned in the esophagus **16** rather than the trachea **18.**

The ventilation system **100** illustrated in FIG. 3 also shows an optional mask **114** with optional venting ports **116.** Variations of the system can include alternate configurations without a mask or with other such devices such as a mouth guard or any other commonly used mounting apparatus. As discussed below, the mask **114** or other mounting apparatus can be used to assist the caregiver in properly orienting the device **100** as it is inserted into the patient. Variations of the device can include a balloon, sponge or any other structure that secures the proximal region of the device to the patient to ensure that gas is directed to the lungs during inhalation. The mask (or other structure as described herein) can include a securing band, tape strip, or temporary adhesive to secure the mask in place on the patient. The mask or similar feature can be used to determine how far to advance the working end **102** into the patient. Alternatively or in combination, the device **100** can include graduated markings **134** to assist the caregiver in properly advancing the device into the patient. The mask can be slidable to adjust the length from the mask to the distal or proximal opening.

FIG. 3 also shows a representative figure of a control system **150** with a number of controls **152** that allow for various device operative sequences, manual controls, or device overrides. For example the system **150** can include manual ventilation controls so that the caregiver can manually adjust inspiration and expiration of the patient. The controls **152** can include a reset or rapid ventilation mode for performing cardio pulmonary resuscitation. The controls **150** include a continuous airflow or continuous vacuum mode that can assist in clearing debris or bodily fluids from the body passages. The controls also allow caregivers to connect the device **100** directly to an endotracheal tube if the caregiver decides to intubate. In an additional variation, the system can allow for active ventilation consisting of blowing for a period and then sucking for a period through the active lumen in order to increase ventilation efficiency. In some variations, the system is configured so that the ventilation openings, as well as other openings on the tube do not rotate relative to the mask so that caregiver can align the openings with the trachea.

The device shown in FIG. 3 can also include one or more electrodes positioned on the working end. For example, the hub **108** can serve as an electrode and apply electricity to the heart in order to defibrillate a patient out of an irregular rhythm or increase the heart rate or contractility. Additionally, one or more electrodes can be inserted on or embedded in a tube designed to be placed in the patient's mouth, esophagus or trachea. Such placement will be beneficial because it would allow the operator a more direct route of electrical stimulation of the heart compared that the current use of pads placed on the patient's chest. A tube placed in the esophagus would more easily be able to detect a pulse because of the major arteries running parallel to the esophagus. This would allow rescuers to determine a pulse without having to touch the patient. It would also allow an untrained bystander to administer CPR who was not trained on checking for pulsed. In addition, the pressure sensors, as described below, can be placed on the tube that had been advanced into the patients mouth that may be pressing against major arteries would be able to detect if there was a change in pressure.

In additional variations, the control system **150** can be integrated into one or more parts of the device body **102** rather than being a separate stand-alone box type configuration. In addition, the ventilation system **100** can be optionally configured to work with a defibrillator. Alternate variations of the system **100** can be configured to provide an audible, visual, or tactile sensation to indicate when a caregiver should administer chest compressions.

FIG. 3 also shows the depicted variation of the device **100** as having an optional balloon **132** or other expandable member located on a working end. When used, the balloon can be positioned anywhere along the device adjacent to the distal opening **106.** Alternatively, or in combination, a balloon can be located adjacent to the medial opening.

The various tubing forming the device **100** should be sufficiently flexible so that the device can be navigated through the upper respiratory system. Alternatively, or in addition, portions of the tubing can be constructed to withstand being collapsed by the patient's mouth or teeth. In additional variations the system **100** can be designed such that the distance between the distal opening **106** is adjustable relative to the medial opening **112** and/or the mask **114** (or even moveable relative to the gradiations **134**). A similar variation includes a medial opening **112** that can be adjustably positioned relative to the distal opening **106,** mask **114** and or gradiations **134**

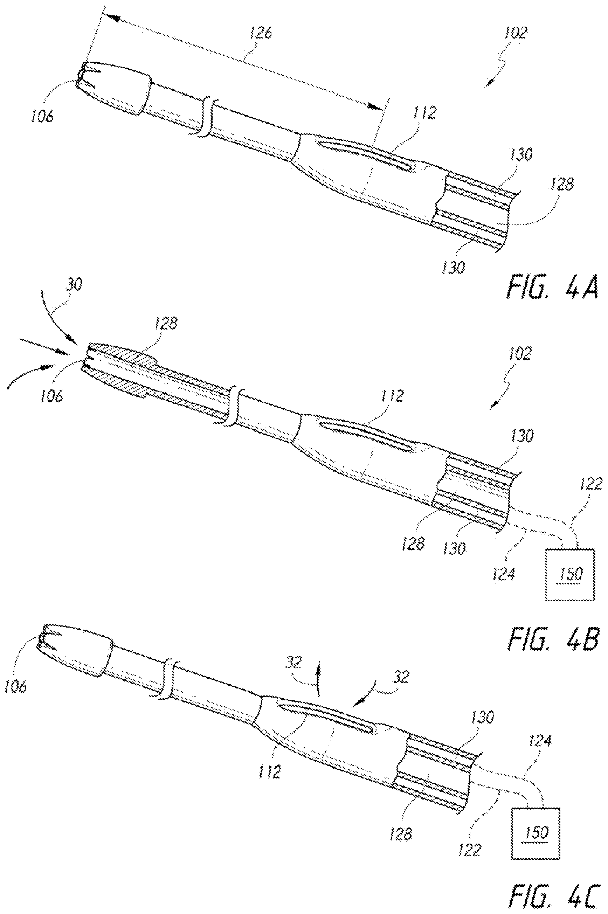

FIGS. 4A to 4C illustrate a partial sectional view of an airway unit or working end **102** of a ventilation device **100** as described herein.

FIG. 4A illustrates a first lumen **128** that is fluidly coupled to a distal opening **106** and a second lumen **130** that is fluidly coupled to the medial opening **112** where the first and second lumens **128** and **130** are fluidly isolated from each other as described above. FIG. 4A also illustrates that the spacing **126** between the distal opening **106** and the medial opening **112** can be selected based on the intended patient. For example, since the medial opening **112** is intended to be positioned in or around the pharynx when the distal opening **106** is positioned in the esophagus or trachea, the spacing **126** can be selected for an individual of average build. In most cases, the working end **102** of the ventilation device **100** will comprise a single use disposable component. Accordingly, the ventilation device **100** can include a number of disposable components having different spacing **126** between the medial **112** and distal **106** openings. For instance, the varying spacing can accommodate infants, toddlers, young children, as well as various body sizes.

FIG. 4B illustrates a partial cross sectional view of the working end **102** of the ventilation device of FIG. 4A. Once the device is properly positioned within the patient, the control unit **150** applies a suction or vacuum through a first fluid path **122,** then through the first lumen **128** and ultimately causing a vacuum at the distal opening **106** as denoted by arrows **30.** In additional variations, the operator or caregiver may choose to clear food or other debris from the patient by delivering air through the first lumen **128** or by attempting to use the suction at the distal opening to remove particles or other bodily fluids. The system **150** shall continue to pull a vacuum through the first lumen **130** for a period of time. If the device **100** is properly positioned within the trachea (as discussed below), the system **150** will begin to ventilate through the first lumen **128.** In other words, the system **100** will begin to cyclically deliver oxygen or other gas from the source **160** and remove carbon dioxide from the patient to properly ventilate the patient's lungs. In this situation, flow is not required through the second lumen **130** and medial opening **112.** Although FIG. 4B shows the first lumen **128** to be located within the second lumen **130** any number of variations can be used. For example, the lumens can be concentric or parallel. Additional variations even allow for the lumens to be in fluid communication where one or more valves determine whether ventilation occurs through the distal opening or through the medial opening. The device can include any number of safety checks to confirm placement of the device doesn't change. For example, once the device confirms placement in the trachea, it can re-perform a check to ensure that it is placed in the trachea over a pre-determined interval. Alternatively, it can perform this check on a sliding scale (e.g., 1^{st} check at 30 second, 2^{nd} check at 2 minutes, 3^{rd} check at 10 minutes, etc.). In an additional variation, the system is designed to provide a safety check to ensure that the suction filter is not clogged causing a misread of position. In such a case the device gives ventilation out the distal port once a vacuum is detected. This ventilation bolus can be small or large. By monitoring vacuum and determining if it is lost during the distal air bolus, the device knows that the seal was at the esophagus and resumes suctioning distally and ventilation through the proximal ports. If the vacuum is not lost during the distal air bolus the device can assume that the filter is clogged and an error signal will indicate for the operator to replace the working end of the device or to check for obstructions.

The system **150** can comprise the mechanism that ventilates and produces suction or a vacuum. Generally, the system **150** is reusable (as opposed to the working end that is generally disposable). The system **150** can be portable, affixed to an ambulance or other emergency vehicle or build within a cart or room. Variations include battery powered devices, pneumatic powered devices, or devices that require a power source (such as an AC outlet).

FIG. 4C illustrates the condition where the distal opening **106** is positioned within the esophagus. In this situation the control unit **150** directs ventilation through the second lumen **130.** As shown by arrows **32,** because the medial lumen **112** is fluidly coupled to the second lumen **130** ventilation **32** takes place at the medial opening **112.**

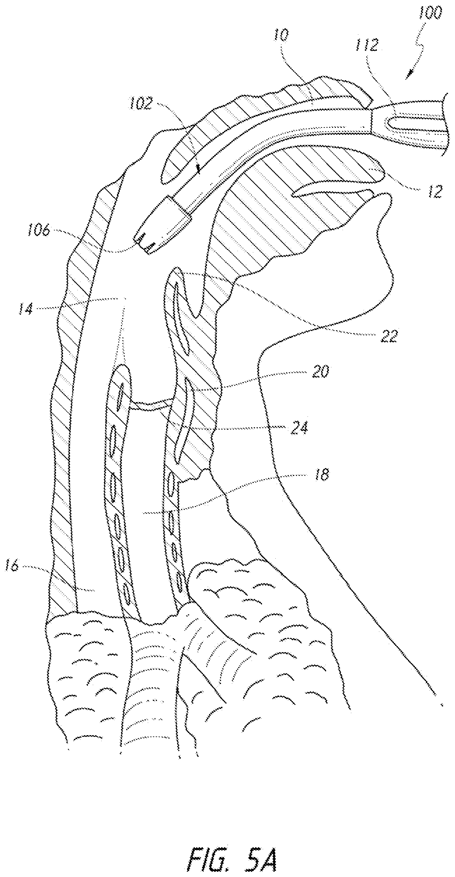

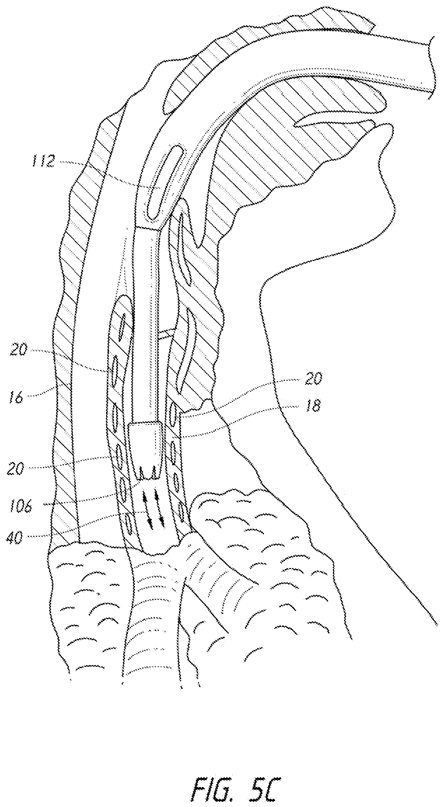

FIGS. 5A to 5E show a representation of the process of ventilating a patient using a ventilation device **100** as described herein.

FIG. 5A illustrates the ventilation device **100** as a caregiver advances the device 100into the oral cavity **10** over the tongue **12** and into the pharynx **14.** At any time during the procedure, the caregiver can manually operate the device to suction fluids, food particles, or other items from the body. As described herein, the caregiver can "blindly" advance the working end **102** into the patient. As a result, the working end **102** will either end up in the esophagus **16** or trachea **18** of the patient.

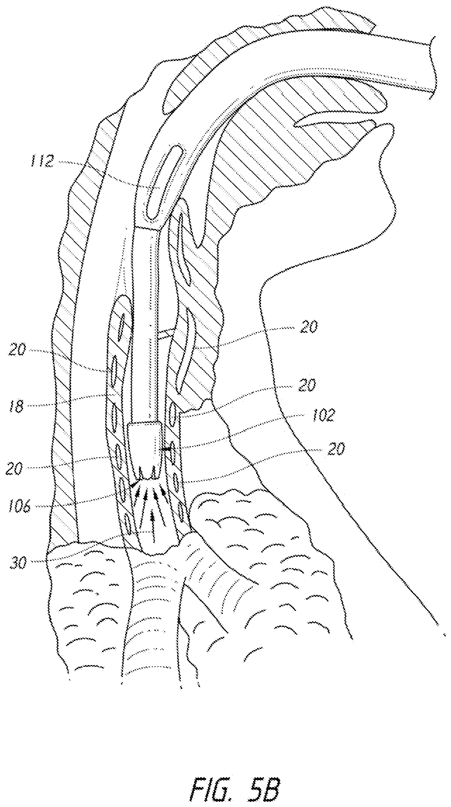

FIG. 5B illustrates the condition where the caregiver advances the working end **102** into a trachea **18** of an individual. Once the caregiver places the device **100,** the caregiver can initiate the control unit **150** to start the process to determine placement of the device **100.** Alternatively, one or more sensors on the device can automatically trigger actuation of the control unit. In either case, the control unit draws a vacuum through the distal opening **106** for a predetermined period of time. The vacuum reduces pressure and draws air within the distal opening **106.** The control unit **150** then assess a state of the device by monitoring the vacuum, airflow, or any other fluid parameter that would indicate whether the walls of the body passage, in this case the trachea **18,** collapsed causing the formation of a vacuum seal. In those cases like FIG. 5B where the device is situated within the trachea, the suction **30** will have little effect on the walls of the trachea **18.** As noted above, the walls of the trachea **18** are reinforced with rings of cartilage **20** that provide structural rigidity of the airway. Because the controller **150** will not detect the formation of a vacuum seal at the distal opening **106** (or within the first lumen) the system registers the distal opening **106** as being properly positioned in the trachea **18** (rather than the esophagus **16**) and, after a pre-determined period of time (e.g., 10-15 seconds), the controller **150** ceases to draw a vacuum and begins to ventilate the patient's lungs by alternating between delivery of the gas from the gas supply **160** and removing carbon dioxide. As a result, the first lumen is used as a ventilation lumen. It will be important for the controller **150** to differentiate changes in vacuum or flow that result from suctioning of fluids or debris. In some variations of the device, the controller **150** is configured to identify formation of a seal when the vacuum builds or flow drops to a sufficient degree such that the device has formed a vacuum seal rather than suctioned fluids or a substance.

The control unit **150** can determine whether or not a seal is formed by measuring strain on a suction motor (or similar apparatus such as a venturi device that produces a vacuum) that causes the negative pressure within the main lumen for suction. If the control unit **150** observes zero or minimal strain on the suction motor after a pre-determined time, then the control unit **150** will use the first lumen as the ventilation lumen.

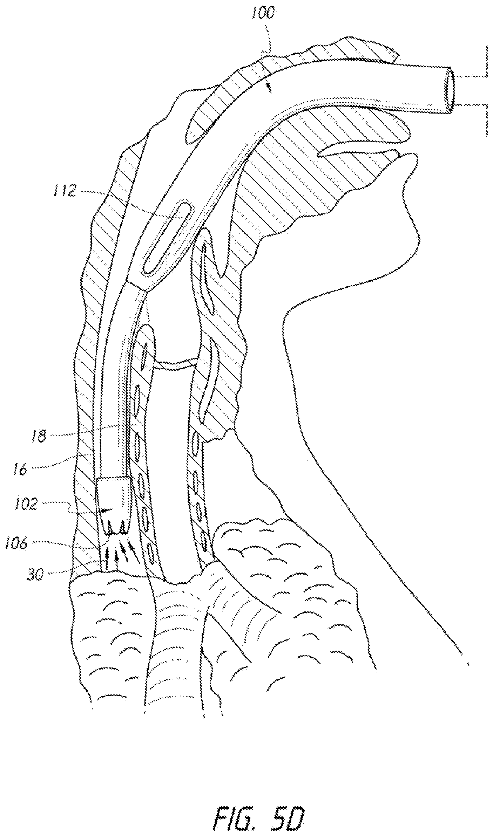

FIG. 5D illustrates a state where the caregiver advances a working end **102** of the ventilation device **100** into an esophagus **16** rather than the trachea **18.** Similarly to the state depicted by FIG. 5B above, once the caregiver positions the device **100,** the caregiver can initiate the control unit **150** to start the process to determine placement of the device **100.** As noted above, additional variations of the device and system can include one or more sensors that can automatically trigger actuation of the control unit.

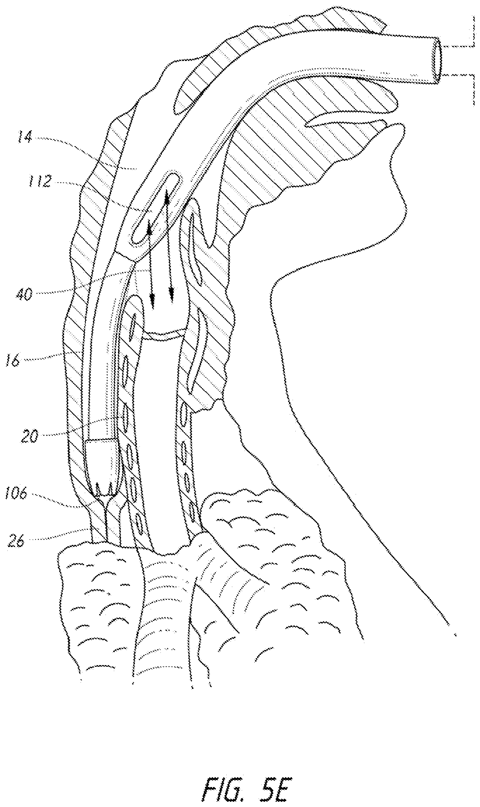

FIG. 5D depicts the state where the control unit **150** pull vacuum through the distal opening **106** for a predetermined period of time. The vacuum reduces pressure and draws air within the distal opening **106**. The control unit **150** then assess a state of the device by monitoring the vacuum, airflow, or any other fluid parameter that would indicate whether the walls of the body passage, in this case the esophagus **16** collapsed. As shown, the walls partially or totally collapse resulting in formation of a vacuum seal at the distal opening **16.** As noted above, muscles form the walls of the esophagus **16.** There is no reinforcing structure in the esophagus as opposed to the cartilage rings in the trachea **18.** The control unit can be configured to monitor the formation of a vacuum seal and if the seal remains for a predetermined period of time, the control unit **150** directs ventilation **40** in and out of the medial opening **112** as depicted in FIG. 5E. As shown and discussed above, the spacing between the distal opening **106** and medial opening **112** can be selected such that the medial opening remains in or near the pharynx **14.** However, variations of the device permit the medial opening to enter the esophagus **16** so long as the opening **112** can continue to ventilate the patient.

Because the control unit **150** will not detect the formation of a vacuum seal at the distal opening **106** (or within the first lumen) the system registers the distal opening **106** as being properly positioned in the trachea **18** (rather than the esophagus **16**) and, after a pre-determined period of time, the control unit **150** ceases to draw a vacuum and begins to ventilate the patient's lungs by alternating between delivery of the gas from the gas supply **160** and removing carbon dioxide. In this situation the device uses the second lumen as a ventilation lumen. One additional benefit of positioning the working end **102** of the device **100** within the esophagus **16** is that the vacuum seal produces an anchoring effect that maintains the device in position. This feature eliminates the need to secure the mask or other feature about the patient's head, neck or face. In addition, if a caregiver inadvertently pulls the device **100** while a seal is formed, the vacuum seal is simply broken and the device releases from the esophagus **16.** This provides a safety improvement over conventional ventilation devices that rely on an expandable balloon, which if pulled, can cause trauma to the patient's airways, vocal cords, or other structures.

In certain variations, the device **100** shall cease ventilating after a period of time and produce suction through the distal opening. Such a step is considered a safety feature in the event that the working end is moved, repositioned, etc.

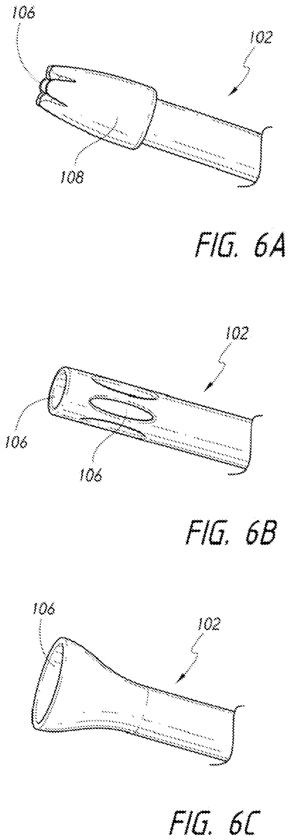

FIGS. 6A to 6C show variations of the working end **102** of a ventilation device as described herein. FIG. 6A illustrates a hub having an opening **106** that is surrounded by a contoured surface. The contoured surface can assist reducing the chance that the distal opening **106** becomes clogged due to food particles or other fluids. This feature also assists in reducing the occurrences that the control unit misreads an opening **106** that is obstructed (with food particles or other bodily fluids) for an opening that formed a seal with the walls of the esophagus. FIGS. 6B and 6C illustrate additional variations of a working end **102** of a ventilation device. In these variations, the working end **102** can be fabricated with or without a hub. FIG. 6B illustrates a straight tube having a plurality of openings **106.** FIG. 6C illustrates a beveled end having an opening **106.**

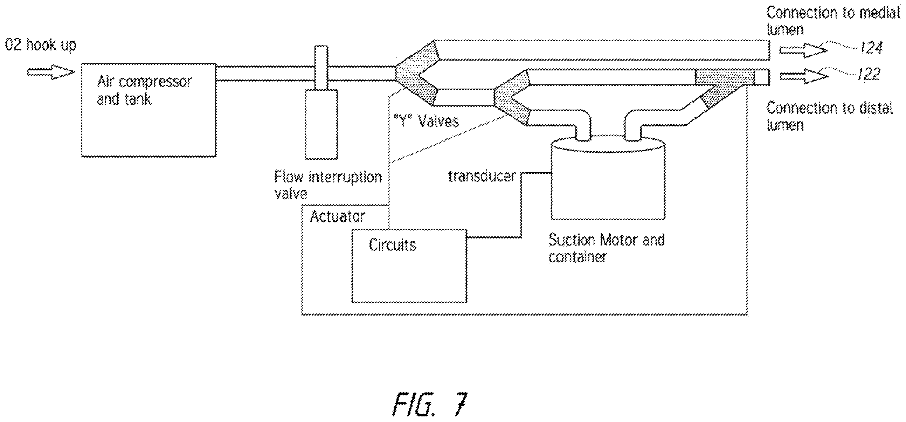

As noted above, the device described herein can be pneumatically driven using compressed gas and valves or electrically controlled. FIG. 7 illustrates a schematic of an electrically powered device using a suction motor, air compressor and circuitry to switch between a first fluid path **122** (ultimately fluidly coupled to a distal opening) and a second fluid path **124** (ultimately fluidly coupled to a medial opening).

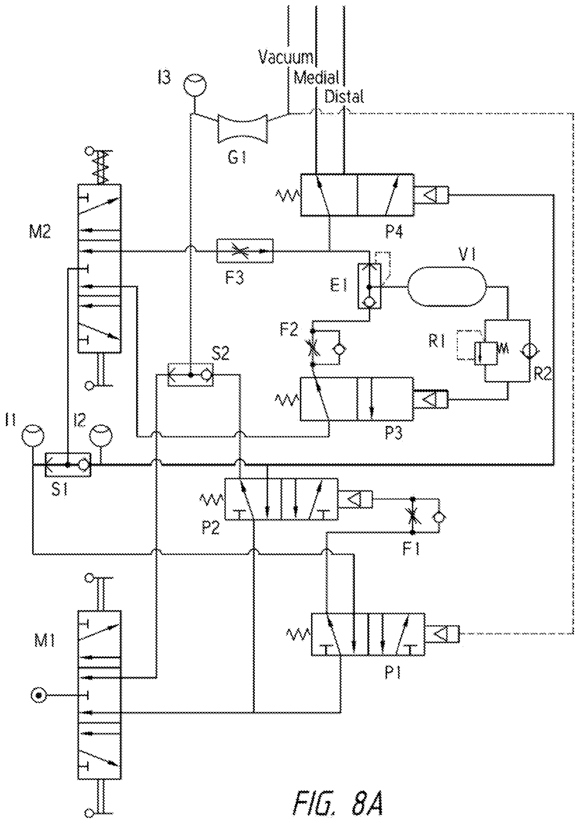

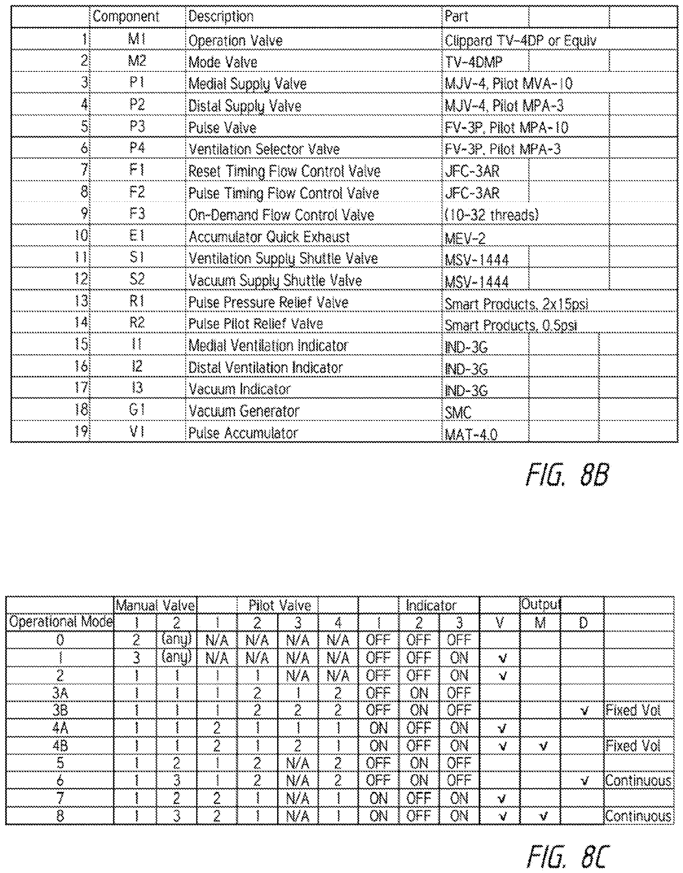

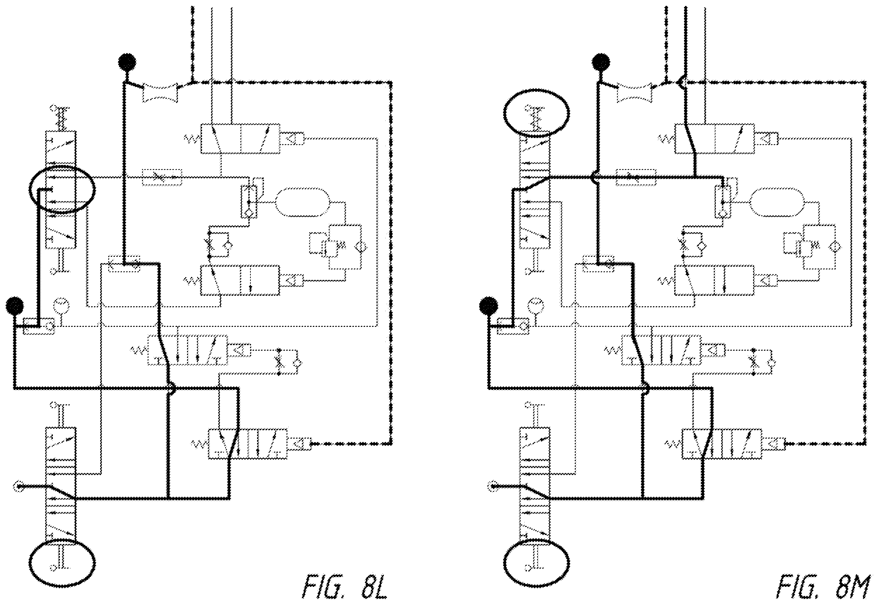

FIG. 8A shows an example of a component schematic for a system as described herein that is pneumatically driven. FIG. 8B provides a list of the components found in FIG. 8A. The valves operate in multiple states based on the conditions discussed above. The following description illustrates an example of the different states of the components found in the component schematic of FIG. 8A.
Medial Supply Valve **P1** (4/2);
State 1 (nominal, spring return): Controls the 15s timing of vacuum supply through Distal Supply Valve **P2;**
State 2 (actuated): Provides supply for medial ventilation;
Pilot Actuation: 10"Hg vacuum
Distal Supply Valve **P2** (4/2)
State 1 (nominal, spring return): Provides supply for Vacuum Generator;
State 2 (actuated): Provides Supply for Distal Ventilation;
Pilot Actuation: 40psi from flow-controlled output of Medial Supply Valve, State 1.
Pulse Valve **P3** (3/2 Normally Open);
State 1 (nominal, spring return): Fills Accumulator volume at flow-controlled rate until set pressure is achieved at inline Relief Valve;
State 2: (actuated): Dumps accumulator volume to Ventilation Selector Valve through quick exhaust;
Pilot Actuation: 5psi from output of inline Relief Valve
Ventilation Selector Valve **P4** (3/2 Fully Ported);
State 1 (nominal, spring return): Routes output of Pulse Valve to Medial Ventilation Output;
State 2: (actuated): Routes output of Pulse Valve to Distal Ventilation Output;
Pilot Actuation: 40psi from output of Distal Supply Valve, State 2
Operation Valve **M1** (Manual Toggle, 3 position, All Detent);
State 1 (toggle down, "ON"): Provides supply for Medial Supply Valve and Distal Supply Valve;
State 2 (toggle centered, "OFF/RESET"): Blocks supply, vents system;
State 3 (toggle up, "VACUUM"): Bypasses all valves, provides supply to Vacuum Generator.
Mode Valve **M2** (Manual Toggle, 3 position, Detent/Detent/Momentary);
State 1 (toggle down, detent, "VENTILATE"): Provides supply for Pulse Valve and Ventilation Selector Valve;
State 2 (toggle centered, detent, "BYPASS"): Blocks supply to Pulse Valve and Ventilation Selector Valve.
State 3 (toggle up, momentary spring return, "ON-DEMAND"): Blocks supply to Pulse Valve, provides continuous flow-controlled supply to Ventilation Selector Valve
The system illustrated by the component schematic of FIG. 8A can have a variety of modes of operation. In one example, as shown by FIG. 8C, the system can include 8 separate modes of operations controlled by the position of various valves and the operation state of a medial supply valve.
Mode 0, where the system is set to an Off position.
M t set to OFF;
Main supply blocked; system vented;

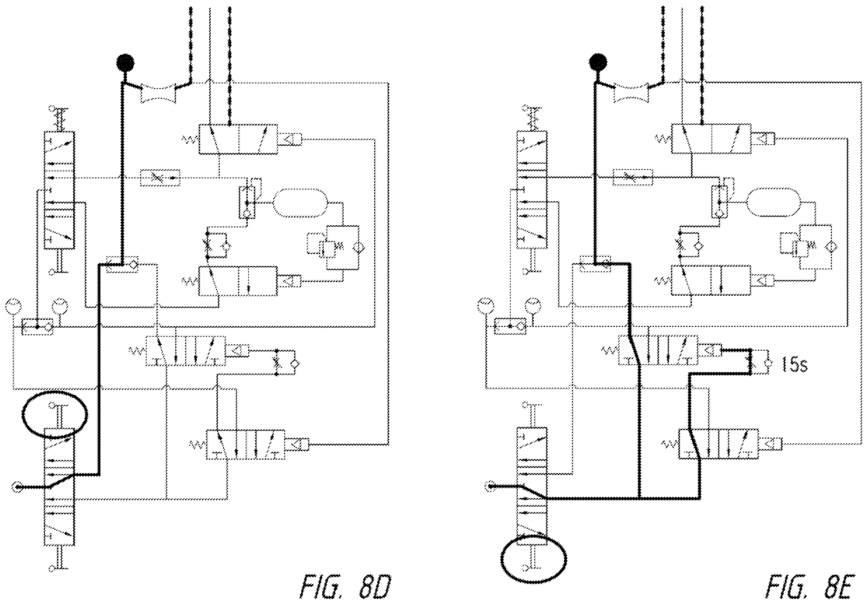

FIG. 8D shows Mode 1, where there is a continuous vacuum applied through the sytem.
M1 set to VACUUM
Ventilation system bypassed; vacuum at Vacuum Output; Vacuum Indicator on
FIG. 8E shows Mode 2, where the system engages in placement detection;
M1 set to ON;
Vacuum at Vacuum Output until P2 pilot activated (15s); Vacuum Indicator on;
In Mode 3, the system engages in ventilation through the distal opening.
M1 set to ON; M2 set to VENTILATE;
No vacuum detected; P2 pilot activated; P4 pilot activated.

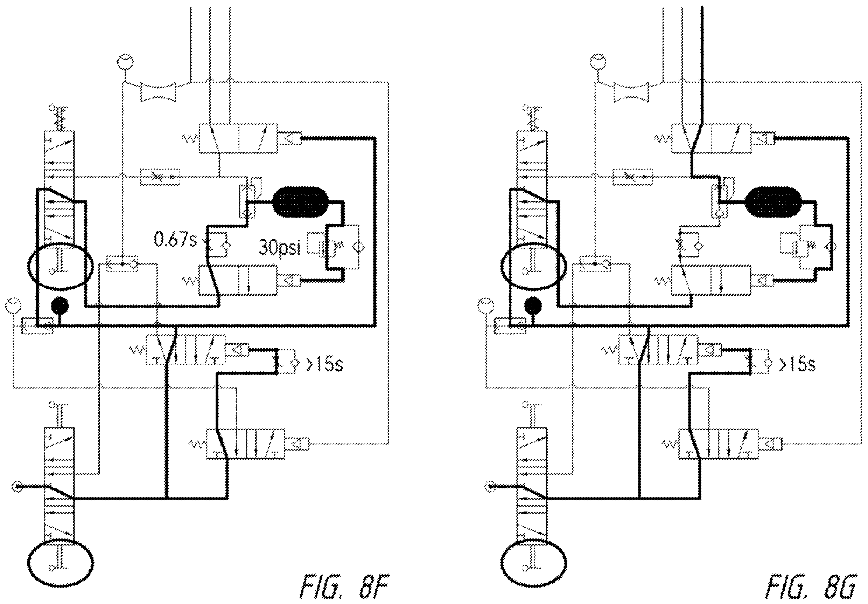

FIG. 8F shows Mode 3A, where an accumulator fills at controlled rate (0.67s) until inline Relief Valve activates (30psi);
Distal Ventilation Indicator on.
FIG. 8G shows Mode 3B: P3 pilot activates, closing P3 and exhausting Accumulator volume through Quick Exhaust to P4; Distal Ventilation Indicator on.
Mode 4 - Medial Ventilation
M1 set to ON; M2 set to VENTILATE
Vacuum detected; P1 pilot activated; vacuum at Vacuum Output.

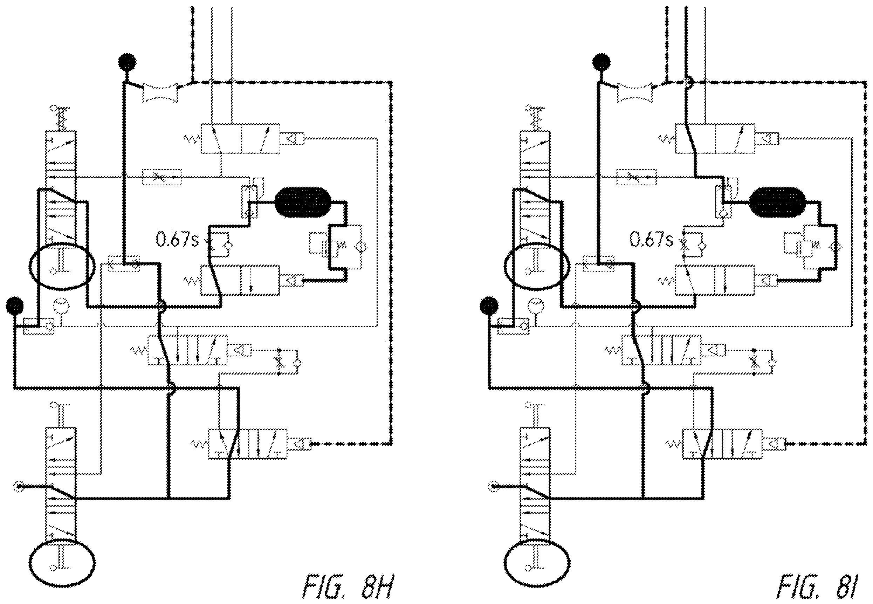

FIG. 8H shows Mode 4A where accumulator fills at controlled rate (0.67s) until inline Relief Valve activates (30psi);
Vacuum Indicator on;
Medial Ventilation Indicator on.
FIG. 8I shows Mode 4B: P3 pilot activates, closing P3 and exhausting Accumulator volume through Quick Exhaust to P4;
Vacuum Indicator on; Medial Ventilation Indicator on.

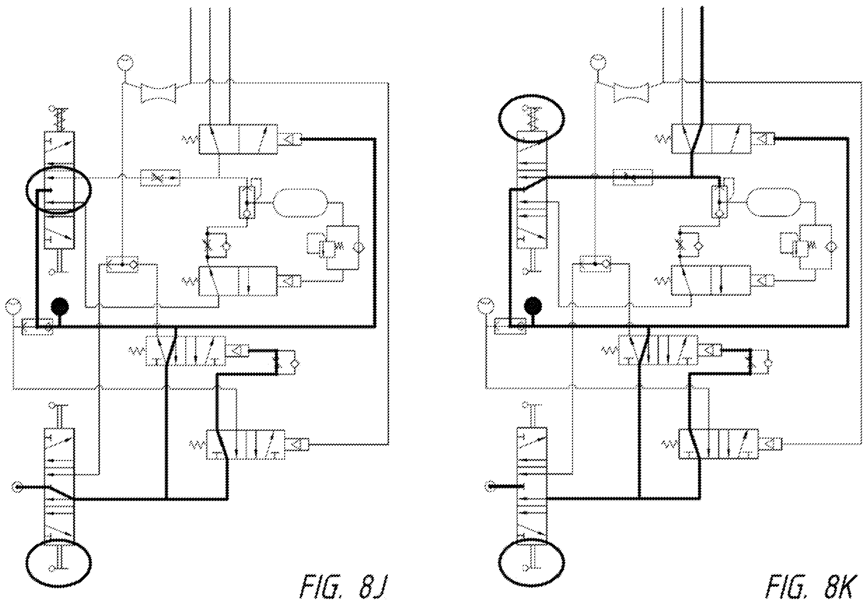

FIG. 8J shows Mode 5 - Ventilation Bypass (Distal);
M1 set to ON; M2 set to BYPASS;
No vacuum detected; P2 pilot activated; P4 pilot activated; supply to P3 & P4 blocked; Distal Ventilation Indicator on.
FIG. 8K shows Mode 6 - On-Demand Ventilation (Distal);
M1 set to ON; M2 set to ON-DEMAND;
No vacuum detected; P2 pilot activated; P4 pilot activated; supply to P3 blocked; continuous flow-regulated flow to P4; Distal Ventilation Indicator on
FIG. 8L shows Mode 7 - Ventilation Bypass (Medial);
M1 set to ON; M2 set to BYPASS;
Vacuum detected; P1 pilot activated; vacuum at Vacuum Output;
supply to P3 blocked;
Vacuum Indicator on;
Medial Ventilation Indicator on
FIG. 8M shows Mode 8 - On-Demand Ventilation (Medial);
M1 set to ON; M2 set to ON-DEMAND;
Vacuum detected; P1 pilot activated; vacuum at Vacuum Output;
supply to P3 blocked;
continuous flow-regulated flow to P4; Vacuum Indicator on; Medial Ventilation Indicator on.

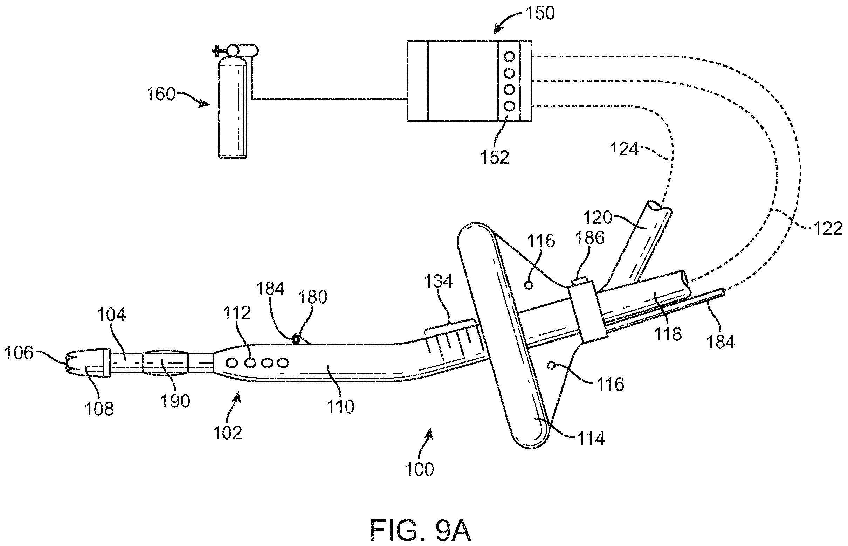

FIG. 9A illustrates another variation of a device useful for providing assisted ventilation with improved outcomes. The features and aspect of the illustrated example can be combined with any of the variations of the devices described herein. Moreover, the features of the devices described herein that improve the effectiveness of assisted ventilation can be used with conventional assisted ventilation devices.

As illustrated, assisted ventilation device **100** includes a working end **102** that is inserted into a patient. The working end can include a distal tubing **104** that contains a first lumen (not shown), which extends through a distal opening **106** of the ventilation device **100** and is in fluid communication with a control unit (also called a ventilator) **150** and/or supply source **160** via one or more proximal tubes **118.** The control unit **150** can also include an apparatus designed to provide suction as well as a collection canister (not shown). As noted above, the device **100** can optionally include an improved control unit **150** that directs suction or applies a vacuum through a first fluid path **122,** which in turn causes a suction or negative pressure at the distal opening **106.** The source **160** can comprise oxygen, air, or any other gas that is desired for ventilation of delivery into the lungs. The source **160** can be nested within physical construct of the controller **150.** However, the source **160** can be optional so that the controller ventilates the patient only using ambient air. FIG. 9A also illustrates the device **100** as including features that allow the assisted ventilation device **100** to deliver ventilation in a manner that improves the efficiency of the assisted ventilation procedure.

For example, the improved device **100** can include one or more structures used to determine a change in the thoracic cavity. Such changes can include physical movement of the tissues within the thoracic cavity, the force applied to the working end **102** of the device **100,** and/or the deflection of any part of the device **100.** Alternatively, or in combination, a change in the thoracic cavity can comprise a change in the fluid environment of the thoracic cavity, including any body passageways that are in fluid communication with the thoracic cavity, e.g., the airway, the esophagus, etc.

FIG. 9A illustrates the device **100** as being able to measure fluid parameters in the thoracic cavity via a sensor **180** located along a portion of the working end **102** of the device. Although the sensor **180** is illustrated on the proximal tubing **110,** the sensor **180** can be positioned along any portion of the device **100** that enables monitoring of the fluid parameters of the thoracic cavity and/or body passage in fluid communication with the thoracic cavity. For instance, the device **100** can include one or more sensors **180** positioned along the distal tubing **104** and/or hub **108.** Moreover, variations of the device include one or more sensors positioned within the device **100.**

The sensor **180** can comprise a pressure sensor, flow sensor, transducer, or similar structure. Alternatively, in additional variations, the sensor **180** can comprise a lumen or passageway having an open end positioned as described above, where the lumen or passageway extends through the device via a sensor tubing **182** that allows the actual fluid parameters to be read by the actual sensor located within the device **100,** tubing **118,** and/or control unit **150.**

The variation illustrated in FIG. 9A also shows a sensor **180** that is not flush with the proximal tubing **110** of the device **100.** As shown, the measurement surface (e.g., the actual sensor or the sensor lumen **184** can be positioned so that tissue adjacent to the device **100** does not obscure or affect the readings of the sensor. However, additional variations of the device **100** include sensors that are flush with the device body. In addition, pressure from the device **100** (e.g., the proximal tube **118** can be used to deliver air to the sensor **180** reduce obstructions from interfering with the measurement of any fluid parameter in the body lumen.

FIG. 9A also illustrates a force detecting component, such as a strain gauge, optic fiber, transducer, or similar force/movement detecting structure that can be located anywhere along the working end **102** of the device **100.** The force detecting component **190** is shown as being on the distal tubing **104,** however, one or more force detecting components **190** can be positioned along any portion of the device as long as the component **190** detects a force applied to the chest via a resulting force being applied to the device through movement of the tissue displaced by assisted chest compression.

The presence of both the sensor **180** and the force detecting component **190** on a single device is for purposes of illustration only. Certain variations of the device can include any combination of force detecting component, sensor, or both.

FIG. 9A also shows the device as including a manual ventilation trigger **186.** In operation, the medical caregiver can use the manual ventilation trigger **186** to manually deliver a bolus of air through the device **100.** Alternatively, or in combination, the manual ventilation trigger **186** can activate the sensor **180** or force detecting component **190** to deliver a bolus of air on demand. Such a feature can be useful if the care giver has obtained a pulse and intends on delivering assisted ventilation alone. Alternatively, the caregiver can use the manual ventilation trigger **186** to deliver a bolus of air through any part of the device in an attempt to clear bodily fluids that might otherwise obstruct the device. The manual ventilation trigger **186** can operate as the device performs the automated assisted ventilation where a bolus of air is delivered at certain period of time. Alternatively, or in combination, the manual ventilation trigger **186** can deliver a bolus of air when the ventilation device **100** (or controller **150**) is placed in a manual-mode.

In certain variations of the device, when initiating the manual trigger **186,** the device be programmed to maintain ventilation through the respective opening that was selected in the automatic mode. For example, if the device is placed in the esophagus, and then switched to manual operation, the control system can maintain suction to ensure that the esophagus closes the distal opening and forms a vacuum so that manual ventilation automatically proceeds through the proximal or medial opening **112.** Likewise, if the device is positioned in the trachea, actuating the device in a manual mode will cause the bolus of air to be expelled from the distal opening of the device.

In one example, the trigger **186** comprises a hollow button, attached to the device and inline with the tubing that connects to the sensor **180.** When the button is pressed it sends an air bolus to the sensor **180** that signals the control system **150** to start assisted ventilation. The volume of air provided by the manual trigger **186** can be preset. Alternatively, air can be delivered until the caregiver releases the trigger **186** to stop the ventilation. In addition, mounting the trigger **186** mounted on the mask **114** is beneficial because it allows the caregiver to ensure the mask **114** is sealed against the patient's face with one or two hands while operating the demand ventilations.

The manual trigger **186** can also operate to with one or more one-way valves (e.g., a flap that allows exhaust of air when the trigger **186** is not pressed). This ensures that there is no excess buildup of pressure in the airway and prevents barotrauma. This also allows spontaneous breathing. When the ventilator is switched to demand ventilation mode the lungs need to be isolated from atmosphere during the inhalation period only. This can be achieved by having the demand ventilation trigger **186** mounted on a flap that is above an opening on the mask. The flap is designed to be opened with when no pressure is being applied to the button, then once pressure is applied to the trigger the flap is sealed against the opening, closing the system and allowing air to inflate the lungs. When the button is released for exhalation the flap is comes off the mask opening allowing air to escape and lungs deflate.

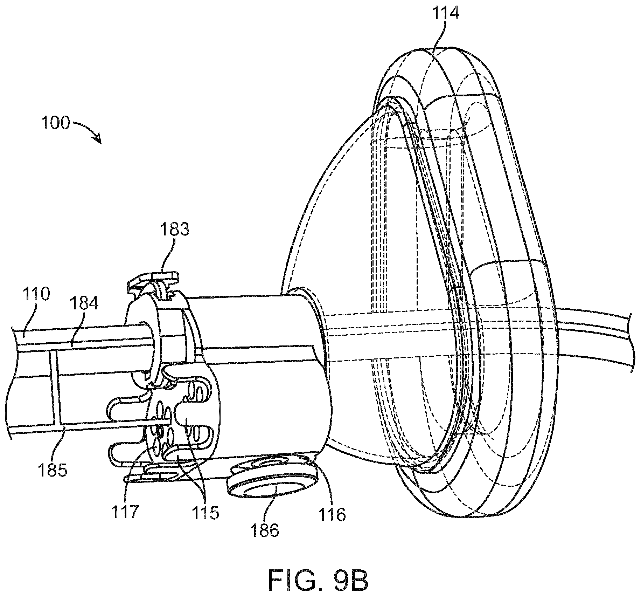

Fig. 9B illustrates a variation of a portion of a device **100** showing a mask **14** having a trigger **186** that is coupled to an exhaust port **116.** In this variation, the mask **114** also includes one or more pressure release valves **117** that will crack or permit flow beyond an established pressure. Such a fail-safe presents unsafe pressurization of the airway by the device. The pressure release valves **117** can be surrounded by protrusions or features **115** that prevent objects from blocking the valves **117.** The device **100** is also shown with an adjustment control **183** that permits movement of the mask **114** along the tube **110.** FIG. 8B also illustrates a tubing **185** that couples the trigger **186** to the sensor lumen **184.** As shown, the sensor lumen **184** can be coupled with a t-fitting or other fluid coupling so that a portion of the lumen is in fluid communication with the trigger **186** as described below.

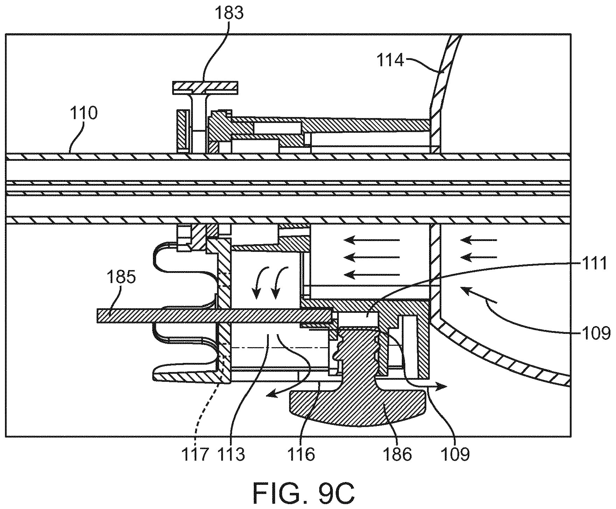

FIG. 9C illustrates a partial cross sectional view of the mask **114** and tubing **110.** As shown, when the mask is positioned against the respiratory opening of the patient (i.e., a mouth or nose), airflow from the expiratory cycle (represented by arrows **109**) flow through a portion of the mask **114** and into a chamber **113** that is in fluid communication with the exhaust lumens **117.** However, in this condition, the trigger **186** is not being pressed against the mask **114** such that the exhaust port **116** remains open allowing airflow **109** to exit the mask. Furthermore, the trigger **186** can be positioned in a shaft having a compressible air volume **111** that is in fluid communication with the tubing **185.** Accordingly, although the mask **114** is pressed against the patient, the exhaust port **116** permits the system to be open (fluidly open).

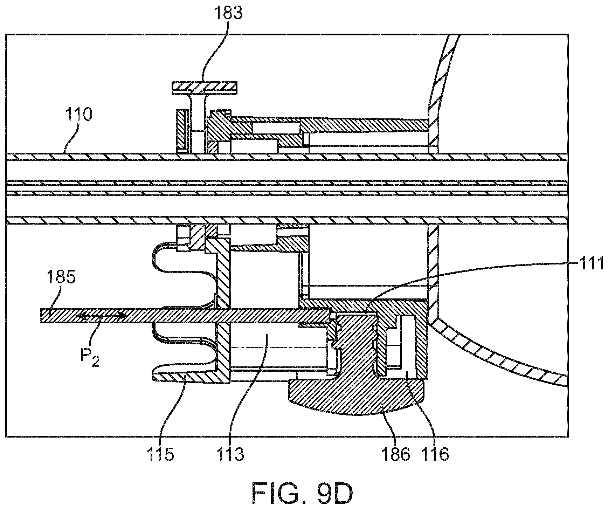

FIG. 9D illustrates a condition where the trigger is pressed or actuated (the trigger can be on a spring return or have elasticity to function as a spring return). Once actuated, the trigger **186** fluidly closes the system by closing the exhaust port **116.** The action of the trigger **186** can also be used initiate a manual bolus of air through the tubing **110.** For example, the trigger **186** can have one or more electrical contacts or switches in region 111 that provides a signal to the control system to deliver a bolus of air. In an additional configuration, actuation of the trigger **186** compresses the volume of air in space **111** causing a pressure increase **P2** in tube **185,** which is coupled to the sensor lumen **184,** this increase in pressure causes the sensor lumen to perform a manual ventilation by delivering a bolus of air through the tubing **110.** Likewise, when the trigger **186** is released, the expansion in volume of region **111** creates a drop in pressure in tubing **185** as well as in the sensor lumen **184,** where the drop in pressure is registered by the sensor to stop ventilation.

In addition to the sensor **180** and/or sensor lumen **184** the device **100** can include any number of additional lumens to provide information to monitoring equipment. For example, the device can include one or more lumens that are fluidly coupleable to a capnograph device. Alternatively, or in combination, the sensor lumen **184** can also allow fluid coupling to a monitoring device. In such a case, the lumens can be coupled to one or more openings (such as **180**) located on the working end of the device.

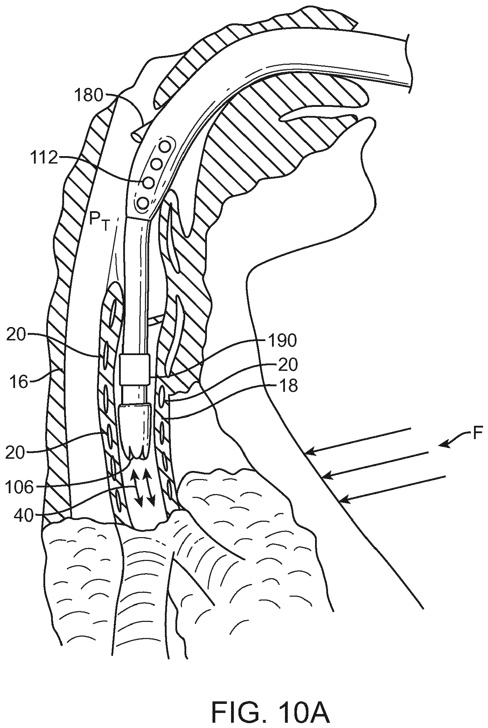

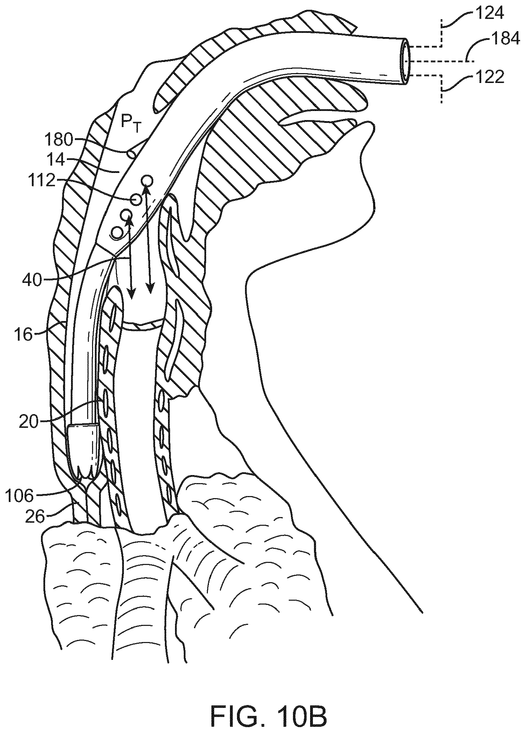

FIG. 10A illustrates an example of the working end **102** of the device **100** when inserted into a body passageway of an individual. In this example, the device is inserted into a trachea **18,** where the device **100** detects that it is in an airway as described above. However, as shown in FIG. 10B, variations of the device **100** can also be positioned in the esophagus **16** using the process described above, which temporally seals the esophagus **16** to deliver air to the respiratory passage **18.**

In either case, the device **100** is configured to begin assisted ventilation by delivering a bolus of air **40** at a pre-determined rate. The device **100** is configured to measure a condition of a thoracic cavity to determine a change in the thoracic cavity, either through pressure within the thoracic cavity as denoted by **PT** or a force **F** applied to the thoracic cavity via chest compressions. In the latter case, the force **F** applied to the chest causes movement of tissue (such as the trachea or other tissue) that can be determined by a force detecting component **190** as discussed above. The detection of a chance in the thoracic cavity by measuring a fluid characteristic such as a change in pressure **PT** is typically measured within a body passageway (such as the trachea **18** or esophagus **16**). Such measurements can include measuring flow rate of air, volume, pressure, etc.

In one variation, the initial or pre-determine rate comprises 100 ventilations per minute (i.e., a bolus of air is delivered 100 times per minute). However, any rate of delivery is within the scope of this disclosure. Upon detecting a change in the condition of the thoracic cavity, typically due to chest compressions, the device **100** will adjust the timing and/or rate of air delivery to achieve an optimum result. For example, the system can deliver a bolus of air upon detecting the chest compression (either by the force measurement or via the fluid sensor measurement). In such a case, the bolus of air increases pressure in the thoracic cavity to serve as an internal chest compression which compresses the heart and lungs from within causing increased blood flow.

In variations of the device, the system monitors for a change in a condition of the thoracic cavity on a continuous basis, or on a delay. In either case, the system can be configured to not respond to a change in the pressure of the thoracic cavity driven by the delivery of the bolus of air. For example, the system can ignore readings during and immediately after the delivery of the bolus of air.

The process of adjusting the delivery of a bolus of air (either by timing and/or rate) in response to a particular phase of the chest compression is intended for use during CPR. However, the assisted ventilation can be accomplished whether using a mechanical compression system or a caregiver performing manual chest compressions.

The alteration of the timing and/or rate is intended to provide a bolus of air with each or a specific number of compression and at a specific phase of the compression of the patient's chest. As noted herein, the ventilations are timed in a way that both increased the efficiency of the chest compression by increasing intrathorasic pressure during the down stroke of the chest compression, which would increase the pressure on the heart thus increasing blood flow. During the up stroke of the compression the a portion of the ventilation could still be given to allow new air enter the alveoli while allowing a portion of the up stroke of the compression to create a negative intrathorasic pressure drawing blood back into the heart and air into the alveoli. This technique also prevents a rescuer from having to pause compressions in order give ventilations, which decreases blood flow and decreases odds of patient's survival.

When using the devices described herein, regardless of whether the device is positioned in the trachea or esophagus, the airway is always opened to the outside environment which greatly reduces, if not eliminates, the chance of barotrauma.

The data generated by the devices described herein regarding the efficiency of the compression regarding depth, rate, recoil time can be analyzed and presented via feedback to the caregiver in order to maximize the efficiency of the compressions. All of this information and be used to increase the efficiency of the compressions and therefore increase blood flow of the patient and increasing patients chance of survival. If using a mechanical compression system the cycle phase could be directly linked to the device **100.**

Furthermore, the system can be configured to return to a pre-determine rate of providing the bolus of air, if at any time chest compression stop/pause. In such a case, the system can monitor the amount of time during which a change in the thoracic cavity is not detected. If no change is detected for a pre-set time, the control unit can reset the rate of assisted ventilation to the initial rate or an alternate rate that is not dependent upon chest compression. In addition, if the patient's pulse resumes, the system can continue to provide assisted ventilation at a pre-determined rate, volume, etc. Alternatively, the system can enter a manual mode where a caregiver can deliver assisted ventilation upon demand (e.g., using the manual trigger button). Furthermore, the system can be configured to check for a patient's pulse and use identification of the pulse to adjust the rate of assisted ventilation or cease assisted ventilation.

The manual trigger allows the caregiver to give a controlled ventilation on demand button may be beneficial once the patient has regained a pulse eliminating the need for external chest compression. As noted above, the device **100** can still continue isolation of the lungs by collapsing the esophagus with suction and/or direct air through the proper lumen into the lungs but changes the ventilation to an air bolus given on demand given by the caregiver. The manual trigger allows the caregiver to start the flow of air to the lungs. Release of the trigger stops the flow of air to the lungs to allow the patient to exhale. Alternatively, a single actuation of the trigger can give a preset amount of air that ventilates the patient.

The system described herein can also be used with conventional rescue devices. For example, the ventilation system can be configured to work with an active chest compression device so that ventilations and chest compressions are timed to increase effectiveness of both the compression and ventilation. The coupling can be mechanical and/or electrical. The ventilation system can also include carbon dioxide sampling so that carbon dioxide levels are outputted via a signal or gas stream to a monitor or other notification means as described herein.

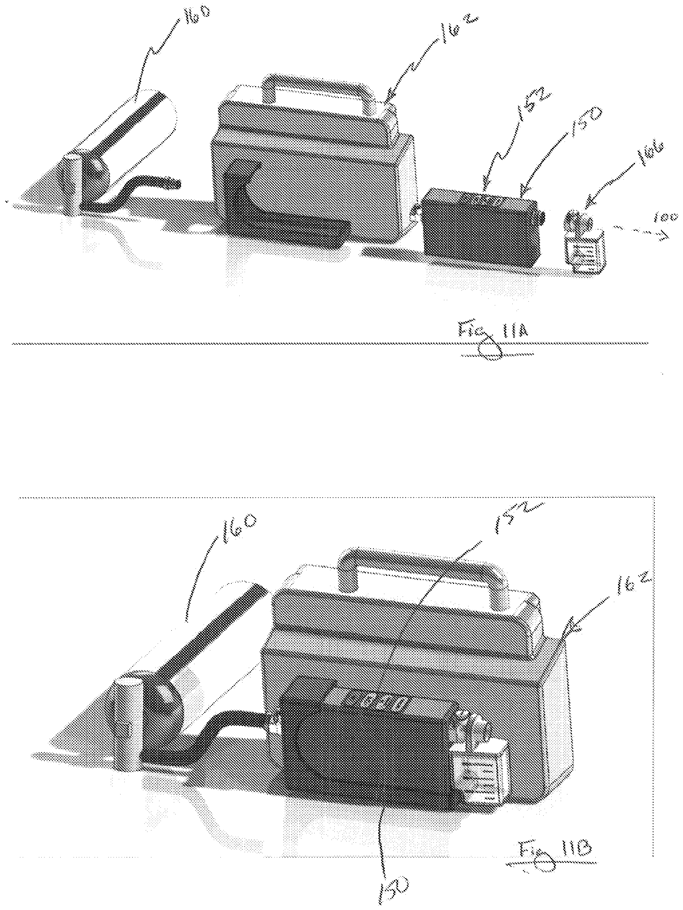

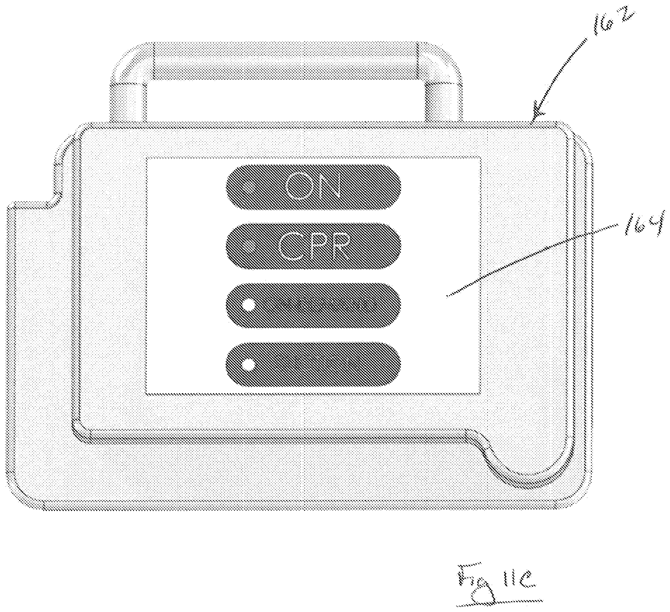

FIGS. 11A and 11B illustrate a variation of a system for artificially ventilating an individual using a source of oxygen, such as those described herein. In the illustrated example, the device **100** is not shown for purposes of clarity in illustration how the control system **150** can be used as a stand-alone unit having electrical control systems having firmware can be controlled through the system interface **152,** or can be integrated or controlled with an external device (e.g., a cardiac monitor, monitor/defibrillator, or other critical care device). As shown, the control unit **150** can be mounted on the external device **162** and coupled to a source of oxygen **160.** As shown in FIG. 11B, once coupled to the external device **162,** the control unit **150** can be operated using the on-board controls **152** or can be controlled via the external device **162** via a wireless or wired connection. In such a case and as shown in FIG. 11C, one or more of the on-board controls **152** of the control unit **150** can be displayed on the control/display **164** of the external device **162.** The variation shown in FIG. 11C illustrates controls for operating the device **100** in a CPR mode, on-demand mode, or suction mode. However, any number of items can be displayed on the control/display **164** and/or on the on-board controls **152.**

For example, the device **100** can display information relating to the phase, rate, efficiency, depth, ratios of chest compression during CPR. Additionally, the device can display information for giving an operator real time feed-back on the efficiency of the assisted compressions via audible or visual feedback as well as information on whether to increase or decrease the speed of manual compressions, or whether to resume chest compressions if pulses are lost or the caregiver stops chest compression for too long.

The device **100** can also be configured with a rechargeable power supply that can be charged when coupled to an external device **162,** or where the connection allows for charging the device **100** via a typical AC power source. In most cases, the control unit **150** will carry a power supply capable of powering the device for a sufficient period of operation and a sufficient stand-by period.

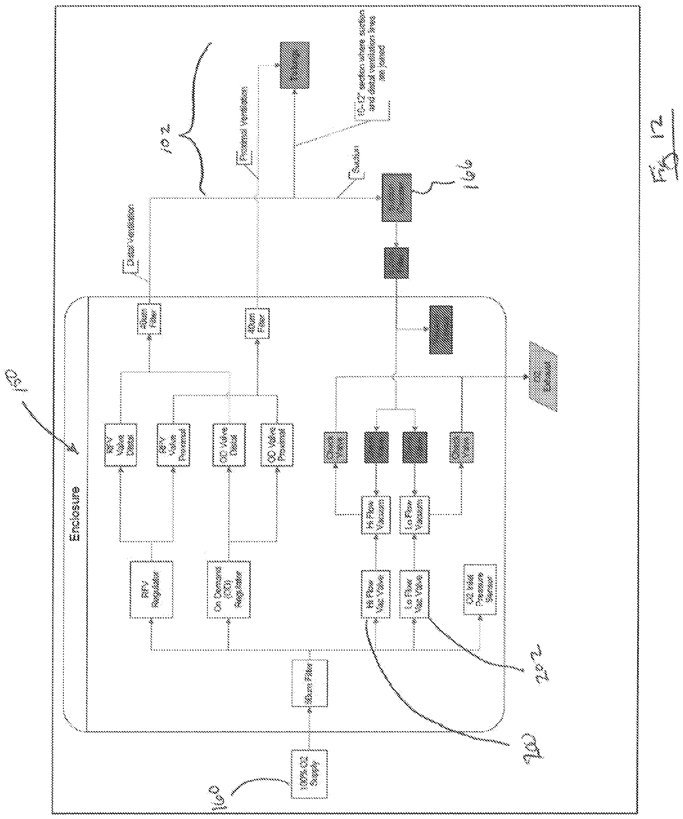

FIG. 12 illustrates an additional improvement to increasing the portability of the control unit **150.** In this configuration, the control unit **150** relies upon the source of oxygen **160** to provide ventilation to the individual as well as to produce the vacuum described above. Accordingly, to extend the source of the oxygen, the device can employ one or more vacuum valves **200, 202** that produce a vacuum as a result of the pressurized flow of oxygen where one vacuum valve operates at a high flow to generate suction. Once a vacuum is established for a certain period of time, the system can switch to a low flow vacuum valve **202** that generates high vacuum with low flow. Such a configuration extends the life of the oxygen supply.

In another variation, the devices described herein can be used to determine ventilation parameters using tubing that accommodates different sizes. For example, having a variety of working ends of different sizes that were coordinated with a Broslow tape for pediatric applications. This way a caregiver could simple select the size airway the Broslow tape recommended and attach to the ventilator. The caregiver would not have to adjust the ventilation parameters because either the authentication process would signal to the ventilator the approximate size of the patient based on the airway selected. Alternatively, the airway itself would reduce the volume, pressure, suction pressure that the patient received. An example of this method would be a narrowing of the ventilation tubing that restricted flow so the volume ventilated over a period of time was less. Another example would be an exhaust valve the dumped excess ventilation volume into the atmosphere, reducing both the volume and pressure for ventilating the patient.

Method for being able to determine the phase, rate, efficiency, depth, ratios of chest compression during CPR by detecting the bending of a tube placed in the patient's mouth, esophagus via various methods. Including but not limited to, strain gauges on tube, fiber optics, air movement sensors. • A method for timing ventilations at a certain phase of the compression to maximize the efficiency of CPR while allowing adequate gas exchange. Using the technology mentioned in the method above attached the ventilator. • A method for continuing ventilations after compressions are stopped or paused. • A method for giving operator real time feed back on the efficiency of rescuers compressions via audible or visual feedback.

Some of the features of the systems described above include: a method for placing electrodes on the tube and pacing the heart via tube placed in the mouth, esophagus or trachea; a method for defibrillating the heart through electrodes placed on a tube in the mouth, trachea or esophagus of a patient; and a method for determining if the patient has a pulse through a tube in the patients mouth, trachea or esophagus.

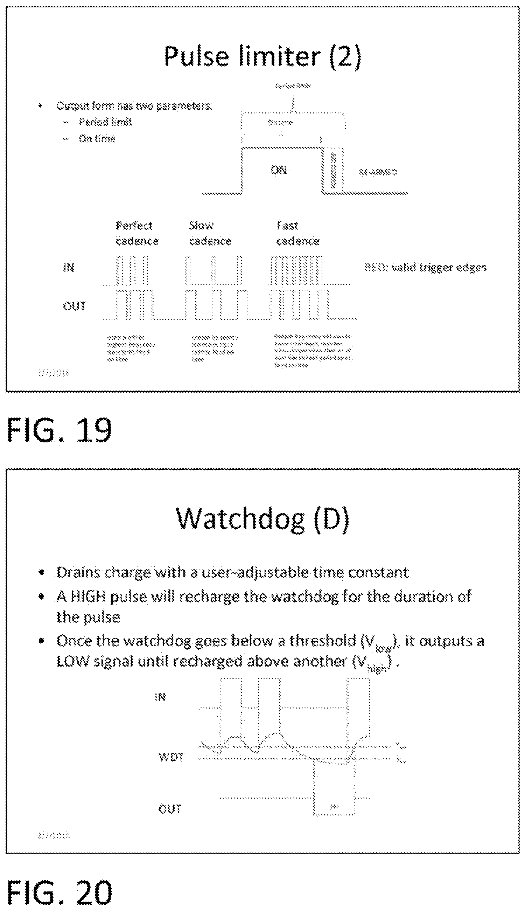

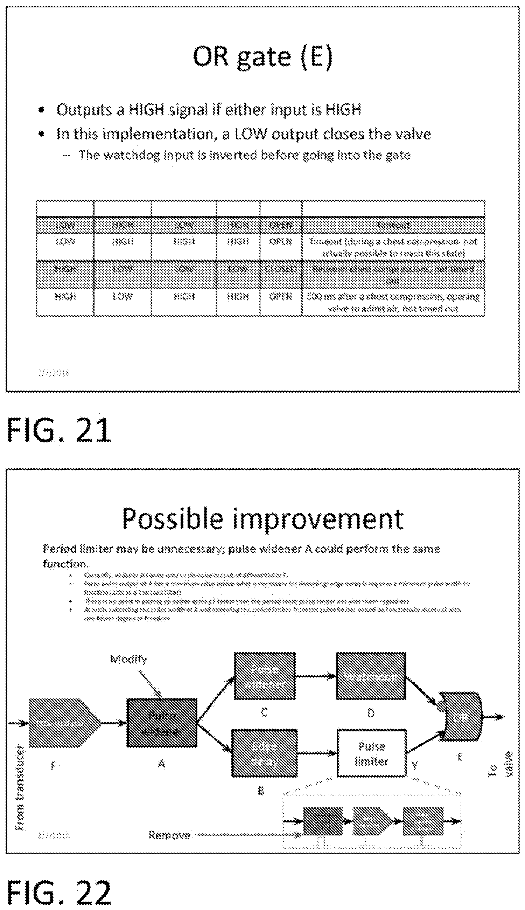

FIGS. 19-28 illustrate an example of the circuitry for sensing the phase, rate, depth and effectiveness of a chest compression with a resistor placed on the tube of an airway placed in the patient's mouth, trachea or esophagus. The information can be relayed to the rescuer or used to signal a valve to time a ventilation. This is only one example of how to implement the invention described above by no way are we limiting other methods mentioned above.

The preceding merely illustrates the principles of the invention.

The scope of the present invention, therefore, is not intended to be limited to the exemplary embodiments shown and described herein. Rather, the scope of present invention is embodied by the appended claims.

It must be noted that as used herein and in the appended claims, the singular forms "a", "an", and "the" include plural referents unless the context clearly dictates otherwise. Thus, for example, reference to "a string" may include a plurality of such strings and reference to "the tubular member" includes reference to one or more tubular members and equivalents thereof known to those skilled in the art, and so forth.

Where a range of values is provided, it is understood that each intervening value, to the tenth of the unit of the lower limit unless the context clearly dictates otherwise, between the upper and lower limits of that range is also specifically disclosed. Each smaller range between any stated value of intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

## Claims

1. A system for artificially ventilating an individual, the system comprising:
a ventilation device (100) configured for insertion within a respiratory opening of the individual and having a working end (102) for positioning within a body passageway of the individual, the ventilation device having a pressure sensor (180) configured to detect pressure changes within the body passageway;
the ventilation device having a control system (150) configured to:
draw suction through a first lumen of the ventilation device to induce collapse of the body passageway and maintain the suction for a period of time;
monitor a fluid parameter to determine whether the body passageway collapses;
automatically ventilate the individual through a second lumen upon detecting collapse of the body passageway and maintain suction to maintain the collapse of the body passageway; and
automatically ventilate the individual through the first lumen upon failure of the monitoring of the fluid parameter to detect collapse of the body passageway;
wherein delivery of a bolus of air during automatically ventilating the individual occurs at a pre-determined rate;
**characterized in that** the system is configured to:
measure a fluid parameter of a thoracic cavity to determine a change in the thoracic cavity resulting from a chest compression; and
upon detecting the chest compression, alter the timing of the delivery of the bolus of air during automatically ventilating the individual, to increase a pressure within the thoracic cavity.

2. The system of claim 1, where delivering the bolus of air into the airway of the individual occurs after a pre-determined delay.

3. The system of claim 1, where the sensor is configured to intermittently detect pressure changes within the body passageway.

4. The system of claim 1, further comprising, after altering the pre-determined rate, the ventilation device (100) resumes delivering the bolus of air at the pre-determined rate if the pressure sensor (180) fails to detect a pressure range within a first period of time.

5. The system of claim 1, where measuring the condition of the thoracic cavity comprises measuring the condition of the thoracic cavity using the ventilation device (100).

6. The system of claim 1, where measuring the condition of the thoracic cavity comprises measuring a change in a compression of a chest of the individual.

7. The system of claim 6, where measuring the change of the compression of the chest of the individual comprises observing a force applied to the ventilation device (100) by the body passageway.

8. The system of claim 6, where measuring the change of the compression of the chest of the individual comprises observing a deflection of the chest of the individual using one or more sensors on the chest.

9. The system of claim 1, where measuring the condition of the thoracic cavity comprises measuring a state of air flow within the thoracic cavity.

10. The system of claim 1, where the control system (150) is configured to provide a feedback based on measuring the condition of the thoracic cavity.

11. The system of claim 10, where the feedback comprises information to increase or decrease a compression applied to a chest of the individual.

12. The system of claim 1, where altering the timing of the ventilation comprises initiating automatically ventilating of the individual when a pressure increases in the body passageway.

13. The system of claim 1, further comprising at least one electrode on the working end (102) that is configured to electrically stimulating a heart of the individual.

## Patentansprüche

1. System für die künstliche Beatmung eines Individuums, wobei das System umfasst:
eine Beatmungsvorrichtung (100), die dazu konfiguriert ist, in eine Atmungsöffnung des Individuums eingeführt zu werden, und ein Arbeitsende (102) für die Positionierung in einem Körperdurchgang des Individuums aufweist, wobei die Beatmungsvorrichtung einen Drucksensor (180) aufweist, der dazu konfiguriert ist, Druckänderungen in dem Körperdurchgang zu erkennen;
die Beatmungsvorrichtung ein Steuersystem (150) aufweist, das konfiguriert ist, zum:
Anziehen eines Sogs durch ein erstes Lumen der Beatmungsvorrichtung, um einen Kollaps des Körperkanals herbeizuführen und den Sog für eine gewisse Zeitspanne aufrechtzuerhalten;
Überwachen eines Fluidparameters, um zu bestimmen, ob der Körperdurchgang kollabiert;
automatisches Beatmen des Individuums durch ein zweites Lumen, wenn ein Kollaps des Körperdurchgangs erkannt wird, und Aufrechterhalten des Sogs, um den Kollaps des Körperdurchgangs aufrechtzuerhalten; und
automatisches Beatmen des Individuums durch das erste Lumen, wenn die Überwachung des Fluidparameters fehlschlägt, um einen Kollaps des Körperdurchgangs zu erkennen;
wobei die Abgabe eines Luftbolus während der automatischen Beatmung des Individuums mit einer vorbestimmten Rate erfolgt;
**dadurch gekennzeichnet, dass** das System konfiguriert ist, zum:
Messen eines Fluidparameters einer Brusthöhle, um eine Änderung in der Brusthöhle zu bestimmen, die aus einer Brustkorbkompression resultiert; und
beim Erkennen der Brustkorbkompression Ändern des Zeitpunkts der Abgabe des Luftbolus während der automatischen Beatmung des Individuums, um einen Druck innerhalb der Brusthöhle zu erhöhen.

2. System nach Anspruch 1, wobei die Abgabe des Luftbolus in den Atemweg des Individuums nach einer vorbestimmten Verzögerung erfolgt.

3. System nach Anspruch 1, wobei der Sensor dazu konfiguriert ist, intermittierend Druckänderungen innerhalb des Körperdurchgangs zu erkennen.

4. System nach Anspruch 1, ferner umfassend, dass die Beatmungsvorrichtung (100) nach dem Ändern der vorbestimmten Rate die Abgabe des Luftbolus mit der vorbestimmten Rate wieder aufnimmt, wenn der Drucksensor (180) beim Erkennen eines Druckbereichs innerhalb einer ersten Zeitspanne fehlschlägt.

5. System nach Anspruch 1, wobei das Messen des Zustands der Brusthöhle das Messen des Zustands der Brusthöhle unter Verwendung der Beatmungsvorrichtung (100) umfasst.

6. System nach Anspruch 1, wobei das Messen des Zustands der Brusthöhle das Messen einer Änderung einer Kompression eines Brustkorbs des Individuums umfasst.

7. System nach Anspruch 6, wobei das Messen der Änderung der Kompression des Brustkorbs des Individuums das Beobachten einer Kraft umfasst, die auf die Beatmungsvorrichtung (100) durch den Körperdurchgang ausgeübt wird.

8. System nach Anspruch 6, wobei das Messen der Änderung der Kompression des Brustkorbs das Beobachten einer Auslenkung des Brustkorbs des Individuums unter Verwendung eines oder mehrerer Sensoren auf dem Brustkorb umfasst.

9. System nach Anspruch 1, wobei das Messen des Zustands der Brusthöhle das Messen eines Zustands des Luftstroms in der Brusthöhle umfasst.

10. System nach Anspruch 1, wobei das Steuersystem (150) dazu konfiguriert ist, eine Rückmeldung auf Grundlage des Messens des Zustands der Brusthöhle bereitzustellen.

11. System nach Anspruch 10, wobei die Rückmeldung Informationen zum Erhöhen oder Verringern einer auf den Brustkorb des Individuums ausgeübten Kompression umfasst.

12. System nach Anspruch 1, wobei das Ändern des Zeitpunkts der Beatmung das Einleiten der automatischen Beatmung des Individuums umfasst, wenn sich ein Druck in dem Körperdurchgang erhöht.

13. System nach Anspruch 1, ferner umfassend wenigstens eine Elektrode an dem Arbeitsende (102), die dazu konfiguriert ist, ein Herz des Individuums elektrisch zu stimulieren.

## Revendications

1. Système pour ventiler artificiellement un individu, le système comprenant :
un dispositif de ventilation (100) configuré pour être inséré dans une ouverture respiratoire de l'individu et ayant une extrémité de travail (102) pour être positionnée dans un passage corporel de l'individu, le dispositif de ventilation ayant un capteur de pression (180) configuré pour détecter les changements de pression à l'intérieur le passage du corps ;
le dispositif de ventilation ayant un système de commande (150) configuré pour :
aspirer à travers une première lumière du dispositif de ventilation pour provoquer un effondrement du passage corporel et maintenir l'aspiration pendant un certain temps ;
surveiller un paramètre de fluide pour déterminer si le passage corporel s'effondre ;
automatiquement ventiler l'individu à travers une seconde lumière lors de la détection d'un effondrement du passage corporel et maintenir une aspiration pour maintenir l'effondrement du passage corporel ; et
automatiquement ventiler l'individu à travers la première lumière en cas d'échec de la surveillance du paramètre de fluide pour détecter l'effondrement du passage corporel ;
dans lequel l'administration d'un bolus d'air pendant la ventilation automatique de l'individu se produit à un débit prédéterminé ;
**caractérisé en ce que** le système est configuré pour :
mesurer un paramètre de fluide d'une cavité thoracique pour déterminer un changement dans la cavité thoracique résultant d'une compression thoracique ; et
lors de la détection de la compression thoracique, modifier le moment de l'administration du bolus d'air pendant la ventilation automatique de l'individu, pour augmenter la pression à l'intérieur de la cavité thoracique.

2. Système selon la revendication 1, dans lequel l'administration du bolus d'air dans les voies respiratoires de l'individu se produit après un délai prédéterminé.

3. Système selon la revendication 1, dans lequel le capteur est configuré pour détecter par intermittence les changements de pression dans le passage corporel.

4. Système selon la revendication 1, comprenant en outre, après avoir modifié le débit prédéterminé, le dispositif de ventilation (100) recommence à administrer le bolus d'air au débit prédéterminé si le capteur de pression (180) ne parvient pas à détecter une plage de pression dans une première période.

5. Système selon la revendication 1, dans lequel la mesure de l'état de la cavité thoracique comprend la mesure de l'état de la cavité thoracique à l'aide du dispositif de ventilation (100).

6. Système selon la revendication 1, dans lequel la mesure de l'état de la cavité thoracique comprend la mesure d'un changement dans une compression de la poitrine de l'individu.

7. Système selon la revendication 6, dans lequel la mesure du changement de compression de la poitrine de l'individu comprend l'observation d'une force appliquée au dispositif de ventilation (100) par le passage corporel.

8. Système selon la revendication 6, dans lequel la mesure du changement de compression de la poitrine de l'individu comprend l'observation d'une déviation de la poitrine de l'individu en utilisant un ou plusieurs capteurs sur la poitrine.

9. Système selon la revendication 1, dans lequel la mesure de l'état de la cavité thoracique comprend la mesure d'un état d'écoulement d'air à l'intérieur de la cavité thoracique.

10. Système selon la revendication 1, dans lequel le système de commande (150) est configuré pour fournir une rétroaction basée sur la mesure de l'état de la cavité thoracique.

11. Système selon la revendication 10, dans lequel la rétroaction comprend des informations pour augmenter ou diminuer une compression appliquée à la poitrine de l'individu.

12. Système selon la revendication 1, dans lequel la modification du moment de la ventilation comprend le lancement automatique de la ventilation de l'individu lorsqu'une pression augmente dans le passage corporel.

13. Système selon la revendication 1, comprenant en outre au moins une électrode sur l'extrémité active (102) qui est configurée pour stimuler électriquement le coeur de l'individu.
